(19) Europäisches Patentamt
European Patent Office
Office européen des brevets

(11) EP 2 657 304 B1

(12) EUROPEAN PATENT SPECIFICATION

(45) Date of publication and mention of the grant of the patent:
**07.12.2022 Bulletin 2022/49**

(21) Application number: **11836229.2**

(22) Date of filing: **24.10.2011**

(51) International Patent Classification (IPC):
***C09B 29/10*** *(2006.01)* ***C09B 29/15*** *(2006.01)*
***C09B 69/10*** *(2006.01)* ***C09B 29/22*** *(2006.01)*
***A61F 2/14*** *(2006.01)* ***A61F 2/16*** *(2006.01)*
***B29D 11/00*** *(2006.01)* ***G02B 1/04*** *(2006.01)*
***G02C 7/10*** *(2006.01)* ***G02C 7/04*** *(2006.01)*

(52) Cooperative Patent Classification (CPC):
**C09B 69/106; A61F 2/1659; B29D 11/00894; C09B 29/10; C09B 29/106; C09B 29/22; G02B 1/043; G02C 7/04; G02C 7/108;** A61F 2/14; A61F 2/16

(86) International application number:
**PCT/JP2011/074471**

(87) International publication number:
**WO 2012/057096 (03.05.2012 Gazette 2012/18)**

(54) **AZO DYE, OCULAR LENS MATERIAL, METHOD FOR PRODUCING OCULAR LENS MATERIAL, AND OCULAR LENS**

AZOFARBSTOFF, OKULARLINSENMATERIAL, VERFAHREN ZUR HERSTELLUNG DES OKULARLINSENMATERIALS UND OKULARLINSE

COLORANT AZOÏQUE, MATÉRIAU DE LENTILLES OCULAIRES, PROCÉDÉ DE PRODUCTION DE MATÉRIAU DE LENTILLES OCULAIRES ET LENTILLES OCULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.10.2010 JP 2010239136**

(43) Date of publication of application:
**30.10.2013 Bulletin 2013/44**

(73) Proprietors:
- **Menicon Co., Ltd.**
  **Nagoya-shi**
  **Aichi 460-0006 (JP)**
- **Dainichiseika Color & Chemicals Mfg. Co., Ltd.**
  **Chuo-ku**
  **Tokyo 103-8383 (JP)**

(72) Inventors:
- **WATANABE Tsuyoshi**
  **Kasugai-shi**
  **Aichi 487-0032 (JP)**
- **BABA Masaki**
  **Kasugai-shi**
  **Aichi 487-0032 (JP)**
- **MOTOYAMA Yuya**
  **Kasugai-shi**
  **Aichi 487-0032 (JP)**
- **KONO Hisao**
  **Tokyo 103-8383 (JP)**
- **KOMIYAMA Nakaji**
  **Tokyo 103-8383 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**EP-A1- 2 330 161** **WO-A1-2009/044853**
**WO-A1-2009/044853** **WO-A2-2006/057840**
**CS-B1- 235 634** **DE-A1- 2 448 994**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

GB-A- 1 537 335
JP-A- 1 297 464
JP-A- 51 119 719
JP-A- 53 111 332
JP-A- 63 091 283
JP-A- 2001 337 298
JP-B- 45 012 593
JP-B1- S4 826 370
US-A- 3 043 647
US-A1- 2009 240 329

- S ACHI ET AL: "Macromolecular azo pigments", DYES AND PIGMENTS, vol. 7, no. 5, 1 January 1986 (1986-01-01) , pages 319-340, XP055205514, ISSN: 0143-7208, DOI: 10.1016/0143-7208(86)80001-8

## Description

[TECHNICAL FIELD]

The present invention relates to an azo colorant suitably used for coloring ophthalmic lenses such as contact lenses and artificial corneas, an ophthalmic lens material colored with the colorant and a method for producing the ophthalmic lens material, and an ophthalmic lens.

[BACKGROUND ART]

Conventionally, in order to prevent colored articles from color fading, colorants having greater color fastness with colorant materials to be colored have been developed. With respect to ophthalmic lenses such as contact lenses and intraocular lenses in particular, preventing elution of the colorant during boiling, and immersion in water or an organic solvent, i.e., color loss, as generally referred to, has been regarded to be important. Accordingly, a method of coloring has been investigated in which color fastness of a colorant with other monomer component in an ophthalmic lens material is enhanced in a production process of the ophthalmic lens material by: copolymerizing a monomer component for an ophthalmic lens with a polymerizable colorant; or mixing a polymeric colorant obtained from a polymerizable colorant with a monomer component for an ophthalmic lens and the like to permit polymerization. An ophthalmic lens manufactured using the ophthalmic lens material colored by such a method is uniformly colored, accompanied by less decolorization and/or discoloration resulting from elution of the colorant, and the like, and is superior in durability against light and/or chemical agents.

In regard to such a polymerizable colorant and a method for coloring an ophthalmic lens material, for example, (1) a polymeric azo colorant having a yellow-coloring moiety and a polymerizable group, and enabling an ophthalmic lens to be colored yellow is disclosed in Japanese Unexamined Patent Application (Translation of PCT Application), Publication No. 2007-505171, Japanese Unexamined Patent Application, Publication No. 2003-84242, and (2) an azo colorant having one or a plurality of polymerizable group(s) and enabling coloring to give various hues is disclosed in the pamphlet of PCT International Publication No. 2009/044853.

Polymerizable light absorbing azo dyes are disclosed in WO 2006/057840 A2 useful as monomers in the formation of devices such as, but no limited to ocular lenses. Specifically, intraocular lenses (IOL) are disclosed wherein one or more of the light absorbing dye is covalently bonded to other structural polymers though ethylene unsaturated groups. The resulting IOLs posses light absorbing properties without significant amounts of free (un-bound) azo dye molecules present in the final structural polymer matrix.

US 2009/0240329 A1 discloses yellow azo dyes for intraocular lenses.

S.S. Achi and T.W.J. Apperley (Macromolecular Azo Pigments, Deyes and Pigments 7 (1986) 319-340) disclose bis-amino azo colours and a method for producing them.

However, since the polymeric azo colorant (1) is a yellow colorant, applicability thereof is limited. On the other hand, although the azo colorant (2) permits coloring to give various hues, coloring of the resultant ophthalmic lens and the like may be nonuniform due to insufficient copolymerizability with the monomer component for an ophthalmic lens, and insufficient miscibility with the monomer component for an ophthalmic lens, and the like, whereby further improvement has been still desired.

[PRIOR ART DOCUMENTS]

[PATENT DOCUMENTS]

Patent Document 1: Japanese Unexamined Patent Application (Translation of PCT Application), Publication No. 2007-505171
Patent Document 2: Japanese Unexamined Patent Application, Publication No. 2003-84242
Patent Document 3: PCT International Publication No. 2009/044853, pamphlet

[SUMMARY OF THE INVENTION]

[PROBLEMS TO BE SOLVED BY THE INVENTION]

The present invention was made in view of the foregoing circumstances, and an object of the invention is to provide an azo colorant being superior in elution resistance from an ophthalmic lens material and in miscibility with a

monomer for an ophthalmic lens, capable of coloring to give various hues, and further provide an ophthalmic lens material that achieves reduced elution of a colorant and is uniformly colored, as well as a method for producing an ophthalmic lens material, and an ophthalmic lens.

[MEANS FOR SOLVING THE PROBLEMS]

An aspect of the present invention made for solving the foregoing problems provides a monoazo colorant for coloring an ophthalmic lens represented by the following general formula (1) :

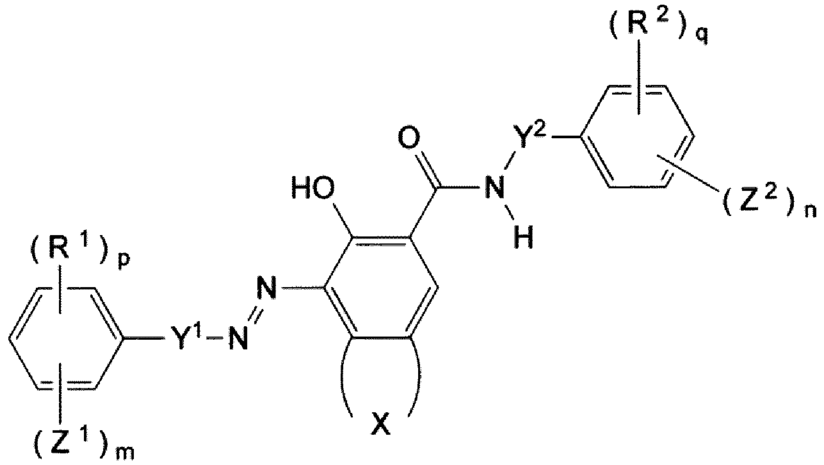

(1)

in the formula (1), X represents a group of atoms that forms a naphthalene ring, an anthracene ring or a benzocarbazole ring together with a benzene ring linking to X; $Y^1$ and $Y^2$ each independently represent a single; $Z^1$ and $Z^2$ each independently represent at least one group selected from the group consisting of groups represented by the following formulae (i) to (vii):

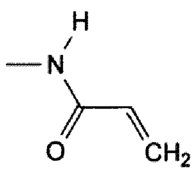

( i )

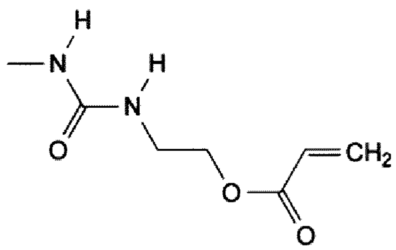

( ii )

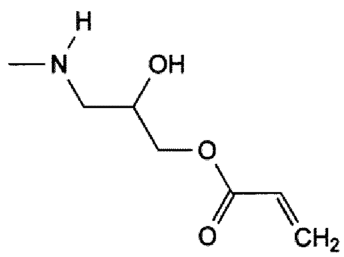

( iii )

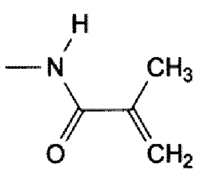

( iv )

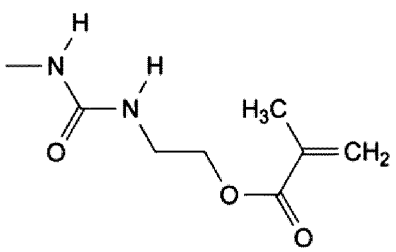

( v )

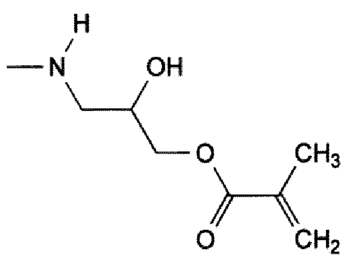

( vi )

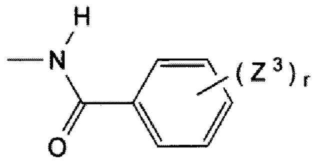

( vii )

in the formula (vii), $Z^3$ represents at least one group selected from the group consisting of groups represented by the above formulae (i) to (vi);

$R^1$ and $R^2$ each independently represent at least one group selected from the group consisting of, an alkyl group having 1 to 5 carbon atoms, and an alkoxy group having 1 to 5 carbon atoms; m, n and r are each independently an integer of 0 to 2, wherein m, n and r are defined such that the total number of the groups represented by the above formulae (i) to (vi) is no less than 2 and no greater than 4; and p is an integer of 2 and and q is an integer of 0 to 5, wherein the sum of p and m, and the sum of q and n are each no greater than 5, wherein in a case where m, n, r, p and q are each no less than 2, a plurality of $Z^1$s, $Z^2$s, $Z^3$s, $R^1$s and $R^2$s are each independently as defined above.

According to the monoazo colorant, since the colorant per se has a plurality of polymerizable groups, a chemical bond can be more efficiently formed with a monomer for an ophthalmic lens having a polymerizable group, and the like. Therefore, according to the monoazo colorant, even when the resultant colored lens is boiled or immersed into water and/or an organic solvent, the colorant is less likely to be eluted from the lens, thereby enabling the lens to be prevented from color loss, as generally referred to.

In addition, various hues can be provided by appropriately adjusting the substituent since the monoazo colorant has the skeleton described above. Furthermore, the monoazo colorant has superior miscibility with the monomer for an ophthalmic lens, due to having the structure described above. Therefore, the monoazo colorant enables an ophthalmic lens material and an ophthalmic lens obtained therefrom to be uniformly colored to give various hues.

In the monoazo colorant, it is preferred that $Y^1$ and $Y^2$ each independently represent a single bond or a divalent organic group having no greater than 20 carbon atoms in which a conjugated system is formed between atomic bonds. According to the monoazo colorant, miscibility with the monomer for an ophthalmic lens is further improved due to $Y^1$ and $Y^2$ having the structure described above, and the monoazo colorant can be easily adjusted so as to give various hues.

The ophthalmic lens material according to another aspect of the present invention is produced from a composition containing the monoazo colorant and a monomer for an ophthalmic lens having a polymerizable group, and thus contains a copolymer of the monoazo colorant and the monomer for an ophthalmic lens.

Due to containing the monoazo colorant, the ophthalmic lens material has high solubility in other monomer for an ophthalmic lens and great color fastness with the other monomer for an ophthalmic lens, whereby uniformly coloring is enabled accompanied by superior elution resistance of the colorant.

The monomer for an ophthalmic lens preferably includes a compound having a polydimethylsiloxane structure. Such a type of the lens material allows solubility of the colorant in the monomer to be further increased, and color fastness of the colorant with the monomer for an ophthalmic lens to be improved, whereby a uniformly colored ophthalmic lens material that is superior in elution resistance can be provided. In addition, according to the lens material, oxygen permeability is improved by providing the monomer for an ophthalmic lens having a polydimethylsiloxane structure as a composition.

It is preferred that the polydimethylsiloxane structure and the polymerizable group are present via a urethane bond in the monomer for an ophthalmic lens. When the monomer for an ophthalmic lens has the structure described above in the ophthalmic lens material, elasticity can be improved while maintaining oxygen permeability.

In the ophthalmic lens material, the monomer for an ophthalmic lens preferably includes a (meth)acrylic acid derivative, a styrene derivative, an allyl group-containing compound, a vinyl group-containing compound, an exo-methylene group-containing compound or a combination thereof. Also such a type of the lens material further increases the solubility of the colorant in the monomer, and thus a uniformly colored ophthalmic lens material can be improved.

It is also preferred that the monomer for an ophthalmic lens is hydrophilic. The ophthalmic lens material can be highly hydrous when the monomer for an ophthalmic lens is hydrophilic.

Yet another aspect of the present invention made for solving the foregoing problems provides a method for producing an ophthalmic lens material, the method including the steps of:

(A) preparing a composition for an ophthalmic lens material containing the monoazo colorant, and a monomer for an ophthalmic lens having a polymerizable group;
(B) transferring the composition into a mold;
(C) curing the composition in the mold by heating, irradiation with an ultraviolet ray, a visible ray or a combination

of an ultraviolet ray and a visible ray, or both the heating and the irradiation to obtain a cured matter; and
(D) releasing the cured matter.

According to the production method, since the composition as a material includes the monoazo colorant described above, an ophthalmic lens material that is superior in elution resistance of the colorant, and is uniformly colored with a desired hue can be obtained.
The step (D) (may be also referred to as "releasing step") is preferably carried out without using a medium, while maintaining the cured matter in a dry state. According to the production method, since a medium such as a liquid is not used, necessity of a step of operation such as removing the liquid, etc., from the cured matter released, and the like is obviated, whereby the production process can be simplified.
It is preferred that the production method further includes the steps of: (E) hydrating the cured matter; and (F) removing an unreacted material from the cured matter hydrated. According to the production method, production of a hydrous ophthalmic lens material that is hydrogelatinous, as generally referred to, is enabled.
It is preferred that the step (E) (may be also referred to as "hydrating step") and the step (F) (may be also referred to as "removing step") be concomitantly carried out by immersing the cured matter obtained through the step (D) in water, physiological saline, phosphoric acid buffer or boric acid buffer. According to the production method, these two steps can be concomitantly carried out, whereby the production process can be simplified.
The monomer for an ophthalmic lens in the production method preferably has a polydimethylsiloxane structure, in light of improvement of oxygen permeability of the resultant ophthalmic lens material and elution resistance of the colorant. Additionally, the monomer for an ophthalmic lens is preferably hydrophilic in light of hydrous properties of the resultant ophthalmic lens material.
Moreover, it is preferred that the method for producing an ophthalmic lens material includes further includes the step of (G) subjecting the cured matter released to a surface treatment. When the cured matter released is subjected to a surface treatment, the ophthalmic lens material can be highly functionalized. The step (G) (i.e., the step of subjecting the cured matter released to a surface treatment) preferably involves any one of: exposing the cured matter to a plasma discharging atmosphere; subjecting a surface of the cured matter to surface graft polymerization; and irradiating the cured matter with an ultraviolet ray or a radioactive ray.
The ophthalmic lens according to still another aspect of the present invention is colored with the monoazo colorant. Since the colorant contained is strongly bound to other component for an ophthalmic lens (monomer for an ophthalmic lens, etc.) as described above in the ophthalmic lens, the colorant is less likely to be eluted from the lens itself even when boiled or immersed in water and/or an organic solvent, whereby color loss of the lens can be prevented. In addition, the ophthalmic lens is uniformly colored, and less likely to be deteriorated due to great color fastness of the colorant with the other component of the material, thereby being superior in durability.

[EFFECTS OF THE INVENTION]

As explained in the foregoing, according to the monoazo colorant of an aspect of the present invention, even when the resultant colored ophthalmic lens is boiled or immersed into water and/or an organic solvent, the colorant is less likely to be eluted from the lens owing to a chemical bond to a monomer for an ophthalmic lens, etc., and thus color loss of the lens can be prevented. In addition, since the colorant of another aspect of the present invention is superior in miscibility with a monomer of the ophthalmic lens, uniform coloring is enabled, and coloring with various hues can be easily executed by adjusting with a substituent, etc.
Furthermore, according to the material for an ophthalmic lens and the ophthalmic lens of yet another aspect of the present invention, uniform coloring to give a desired hue is enabled, and the colorant contained is less likely to be eluted, thereby capable of exhibiting superior durability. Still further, according the method for producing a material for an ophthalmic lens of still another aspect of the present invention, such a material for an ophthalmic lens can be produced.

[BRIEF DESCRIPTION OF THE DRAWINGS]

Fig. 1 shows a view illustrating an infrared absorption spectrum of an azo colorant 1 obtained in Example;
Fig. 2 shows a view illustrating a $^1$H-NMR spectrum of the azo colorant 1 obtained in Example;
Fig. 3 shows a view illustrating an infrared absorption spectrum of an azo colorant 2 obtained in Example;
Fig. 4 shows a view illustrating a $^1$H-NMR spectrum of the azo colorant 2 obtained in Example;
Fig. 5 shows a view illustrating an infrared absorption spectrum of an azo colorant 3 obtained in Example;
Fig. 6 shows a view illustrating a $^1$H-NMR spectrum of the azo colorant 3 obtained in Example;

Fig. 7 shows a view illustrating an infrared absorption spectrum of an azo colorant 9 obtained in Example;
Fig. 8 shows a view illustrating an infrared absorption spectrum of an azo colorant 15 obtained in Example;
Fig. 9 shows a view illustrating an infrared absorption spectrum of an azo colorant 18 obtained in Example; and
Fig. 10 shows a view illustrating a $^1$H-NMR spectrum of the azo colorant 18 obtained in Example.

[DESCRIPTION OF EMBODIMENTS]

Hereinafter, the monoazo colorant, an ophthalmic lens material in which the same is used, a method for producing an ophthalmic lens material and an ophthalmic lens of embodiments of the present invention will be described in detail in this order.

Monoazo Colorant

The monoazo colorant of the embodiment of the present invention is represented by the above general formula (1).

The monoazo colorant can be chemically bound to a monomer for an ophthalmic lens having a polymerizable group and the like since the colorant itself has a plurality of (i.e., two to four) polymerizable groups (the groups represented by the above formulae (i) to (vi)). Therefore, according to the monoazo colorant, even when the resultant colored lens is boiled or immersed into water and/or an organic solvent, the colorant is not eluted from the lens, whereby so-called color loss of the lens can be prevented. In addition, since the monoazo colorant can be chemically bound to a monomer having a polymerizable group, etc., in this manner, the ophthalmic lens colored is less likely to be deteriorated, and thus durability of the colored ophthalmic lens can be improved.

Moreover, since the monoazo colorant has the skeleton described above, various hues can be provided by appropriately adjusting the substituent. Furthermore, the monoazo colorant has superior miscibility with the monomer for an ophthalmic lens due to having the structure described above. Therefore, the monoazo colorant enables an ophthalmic lens material and an ophthalmic lens obtained therefrom to be uniformly colored to give various hues.

The substituent which may be included in the naphthalene ring, anthracene ring or benzocarbazole ring formed together with the benzene ring linking to X in the above formula (1) is particularly limit, and is exemplified by an alkyl group, an alkoxy group, an aryl group, an alkenyl group, an amino group, a mercapto group, a hydroxyl group, a carboxyl group, and the like.

The divalent organic group represented by $Y^1$ and $Y^2$ is not particularly limited, and examples thereof include a divalent aromatic hydrocarbon group, a divalent saturated or unsaturated aliphatic hydrocarbon group, a divalent heterocyclic group, -CO-, -O-, -NH- or a group constituted with a combination of these groups, and the like. However, $Y^1$ and $Y^2$ do not include a group that includes an azo group (-N=N-). The divalent aromatic hydrocarbon group, saturated or unsaturated hydrocarbon group, heterocyclic group may have a substituent. The substituent is exemplified by an alkyl group, an alkoxy group, an aryl group, an alkenyl group, an amino group, a mercapto group, a hydroxyl group, a carboxyl group, and the like. These substituents may be selected in accordance with the desired color tone, etc., of the monoazo compound.

$Y^1$ and $Y^2$ preferably represent a single bond or an organic group in which a conjugated system is formed between atomic bonds. When $Y^1$ and $Y^2$ represent a single bond or an organic group in which a conjugated system is formed between atomic bonds, a structure in which the conjugated system of the monoazo colorant of the embodiment of the present invention spreads over two benzene rings bound at right and left sides can be provided. Such an azo colorant enables a colorant having various desired hues to be obtained through adjusting each substituent and the like. In this regard, the organic group in which a conjugated system is formed between atomic bonds includes a vinylene group ($-CH_2=CH_2-$), a carbonyl group (-CO-), a phenylene group ($-C_6H_4-$) and the like in which an unsaturated bond and a single bond form a conjugated system, as well as an amine structure, a group that constitutes a conjugated system with an unshared electron pair such as an amide group, and a group constituted with a combination of these.

Furthermore, in the case in which $Y^1$ and $Y^2$ represent an organic group that constitutes an intramolecular conjugated system, the organic group is further preferably a group having no greater than 20 carbon atoms, and particularly preferably a group having no greater than 10 carbon atoms. When the number of carbon atoms in $Y^1$ and $Y^2$, which serve as a linking group, falls within this range, the monoazo colorant can have a compact structure, whereby miscibility with other monomer for an ophthalmic lens can be further improved.

Preferred group represented by $Y^1$ and $Y^2$ is exemplified by groups shown below.

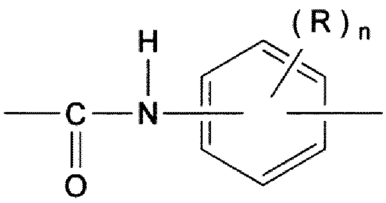

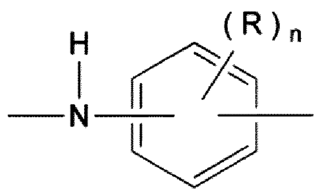

wherein, in the above formulae, R each independently represents an alkyl group having 1 to 5 carbon atoms or an alkoxy group having 1 to 5 carbon atoms; and n is an integer of 0 to 2.

Examples of the halogen atom represented by $R^1$ and $R^2$ in the formula (1) include a fluorine atom, a chlorine atom, a bromine atom, an iodine atom, and the like.

Examples of the alkyl group having 1 to 5 carbon atoms represented by $R^1$ and $R^2$ include a methyl group, an ethyl group, a propyl group, an isopropyl group, a butyl group, a pentyl group, and the like.

Examples of the alkoxy group having 1 to 5 carbon atoms represented by $R^1$ and $R^2$ include a methoxy group, an ethoxy group, a propoxy group, an isopropoxy group, a butoxy group, a pentoxy group, and the like.

Examples of the N,N-dialkylamino group having 1 to 4 carbon atoms represented by $R^1$ and $R^2$ include an N,N-dimethylamino group, an N,N-diethylamino group, and the like.

Examples of the amino group substituted with an aromatic group having 6 to 12 carbon atoms represented by $R^1$ and $R^2$ include a phenylamino group, a diphenylamino group, and the like.

Examples of the alkylamide group having 1 to 5 carbon atoms represented by $R^1$ and $R^2$ include a methylamide group, an ethylamide group, a n-butylamide group, and the like.

Specific examples of the azo colorant include the following compounds.

A compound (hereinafter, may be also referred to as "azo colorant 1") represented by the following formula (1-1):

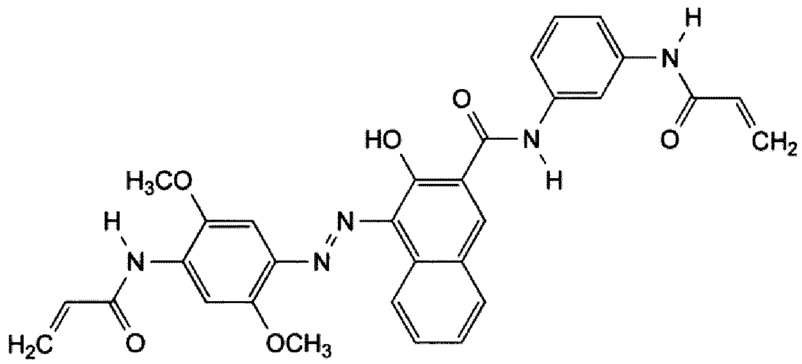

(1 - 1)

A compound (hereinafter, may be also referred to as "azo colorant 2") represented by the following formula (1-2):

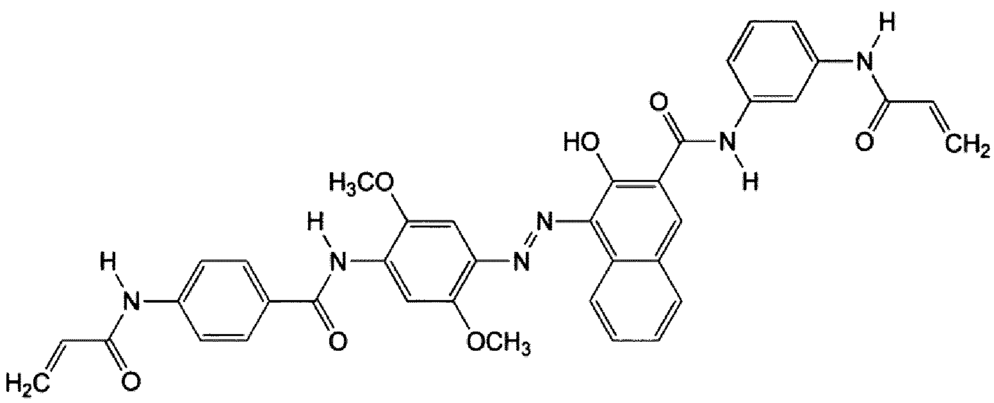

(1 - 2)

A reference compound (hereinafter, may be also referred to as "azo colorant 3") represented by the following formula (1-3) :

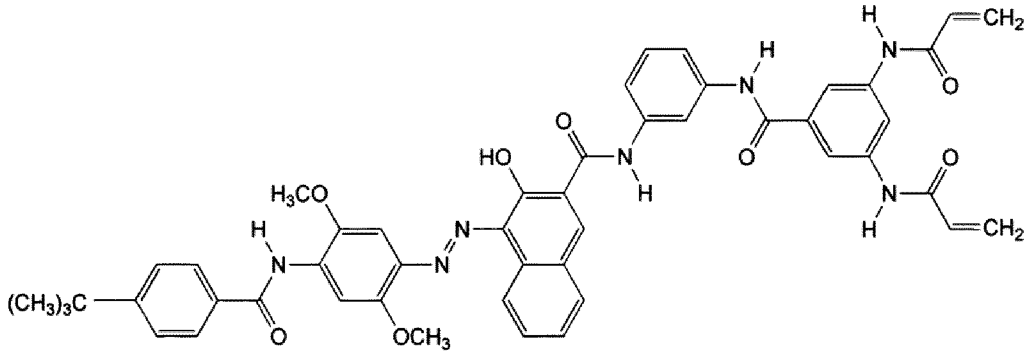

(1-3)

A compound (hereinafter, may be also referred to as "azo colorant 4") represented by the following formula (1-4):

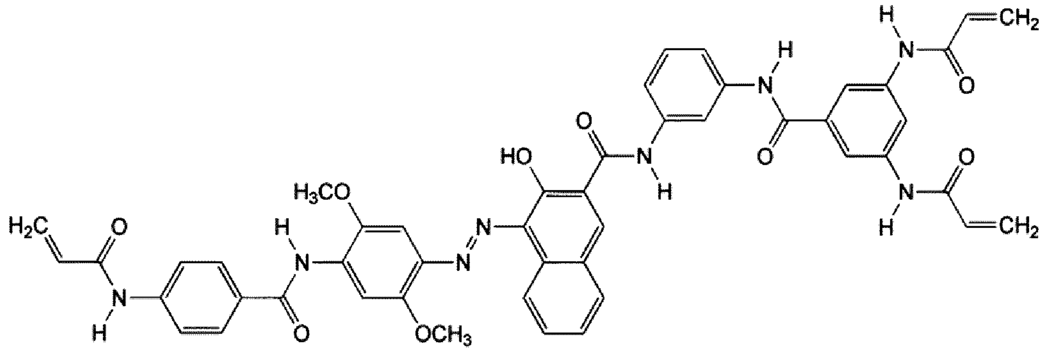

(1-4)

A compound (hereinafter, may be also referred to as "azo colorant 5") represented by the following formula (1-5):

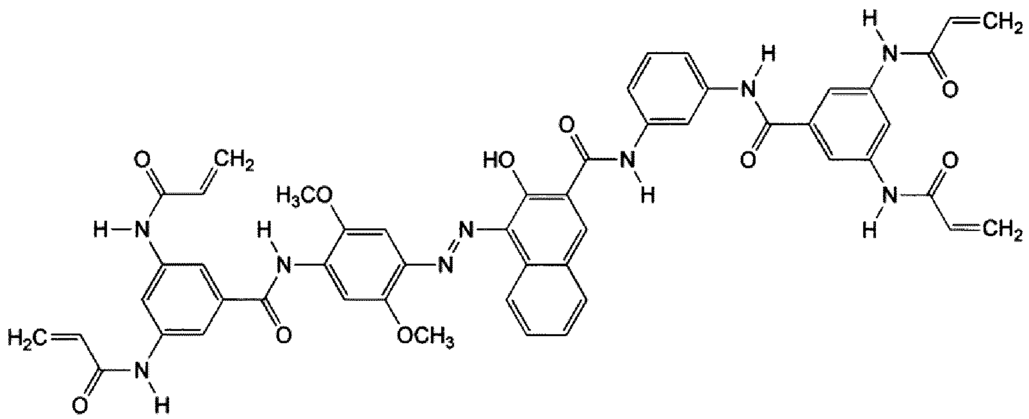

(1-5)

A reference compound (hereinafter, may be also referred to as "azo colorant 6") represented by the following formula (1-6) :

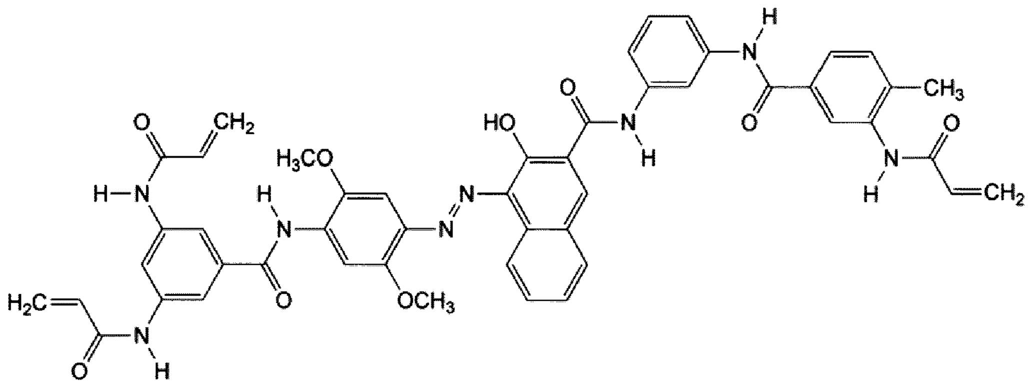

(1-6)

A compound (hereinafter, may be also referred to as "azo colorant 7") represented by the following formula (1-7):

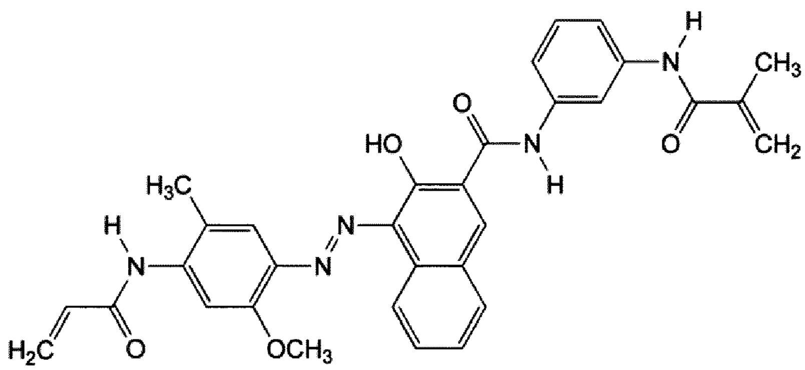

(1-7)

A reference compound (hereinafter, may be also referred to as "azo colorant 8") represented by the following formula (1-8) :

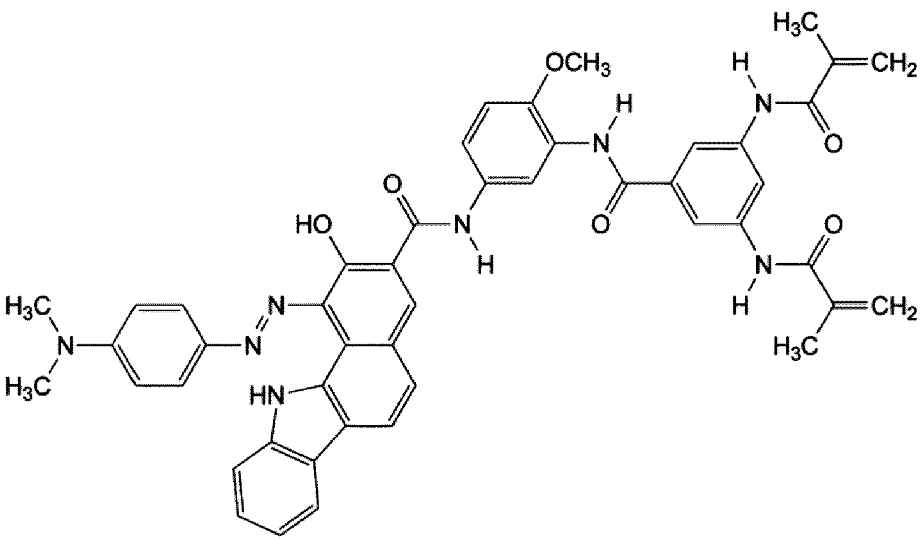

(1-8)

A reference compound (hereinafter, may be also referred to as "azo colorant 9") represented by the following formula (1-9) :

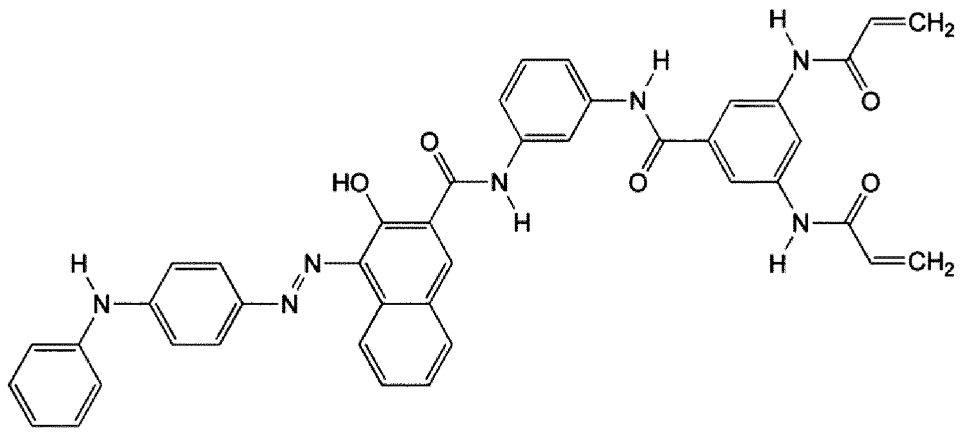

(1-9)

A reference compound (hereinafter, may be also referred to as "azo colorant 10") represented by the following formula (1-10):

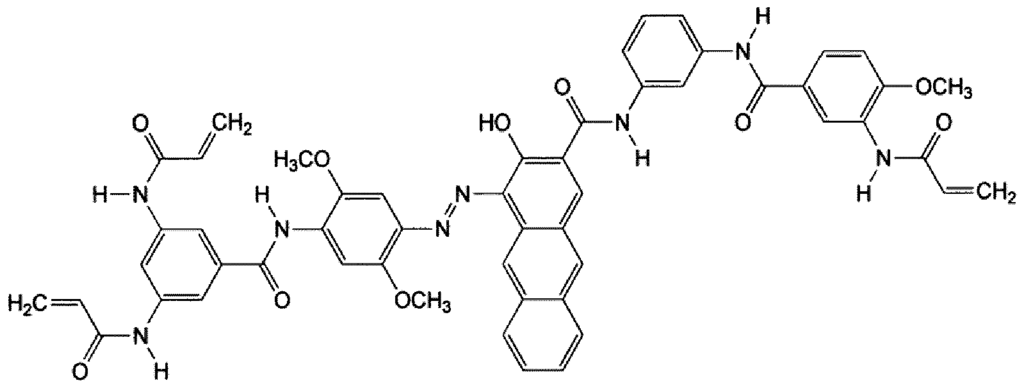

(1-10)

A compound (hereinafter, may be also referred to as "azo colorant 11") represented by the following formula (1-11):

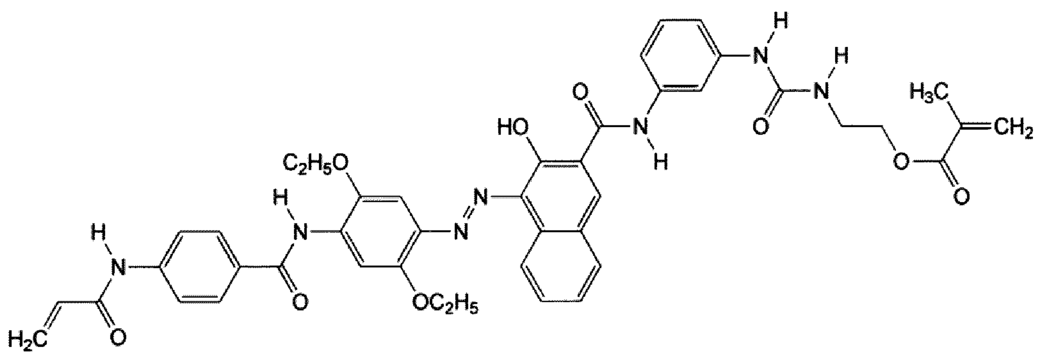

(1-11)

A reference compound (hereinafter, may be also referred to as "azo colorant 12") represented by the following formula (1-12):

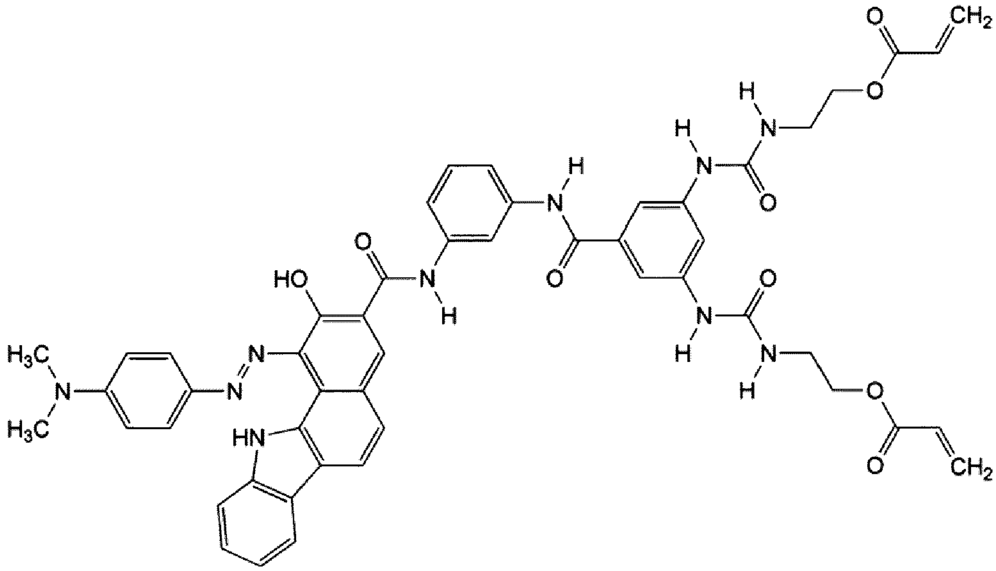

( 1 - 12 )

A reference compound (hereinafter, may be also referred to as "azo colorant 13") represented by the following formula (1-13) :

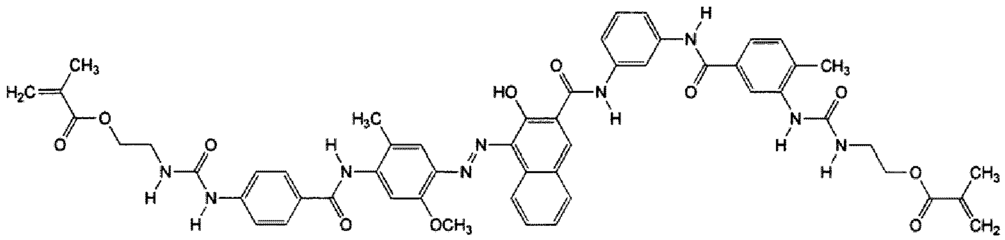

( 1 - 13 )

A reference compound (hereinafter, may be also referred to as "azo colorant 14") represented by the following formula (1-14) :

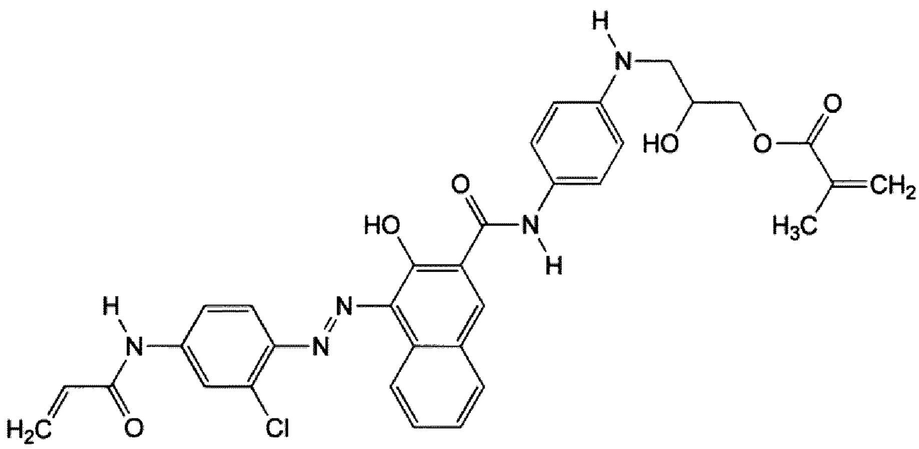

( 1 - 14 )

A reference compound (hereinafter, may be also referred to as "azo colorant 15") represented by the following formula (1-15):

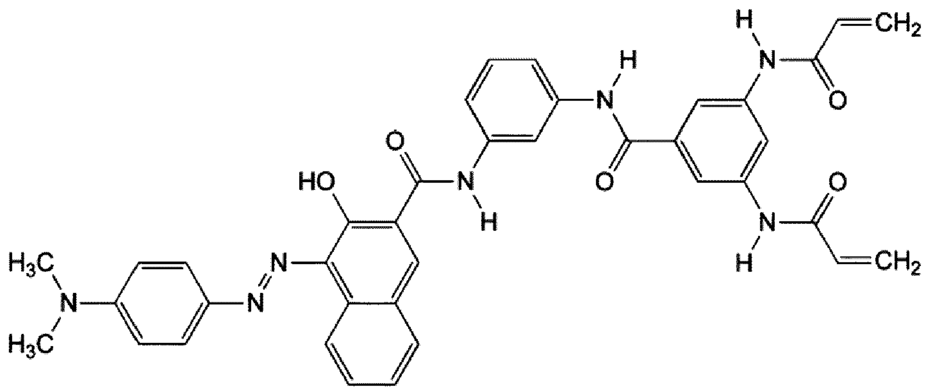

( 1 - 15 )

A reference compound (hereinafter, may be also referred to as "azo colorant 16") represented by the following formula (1-16):

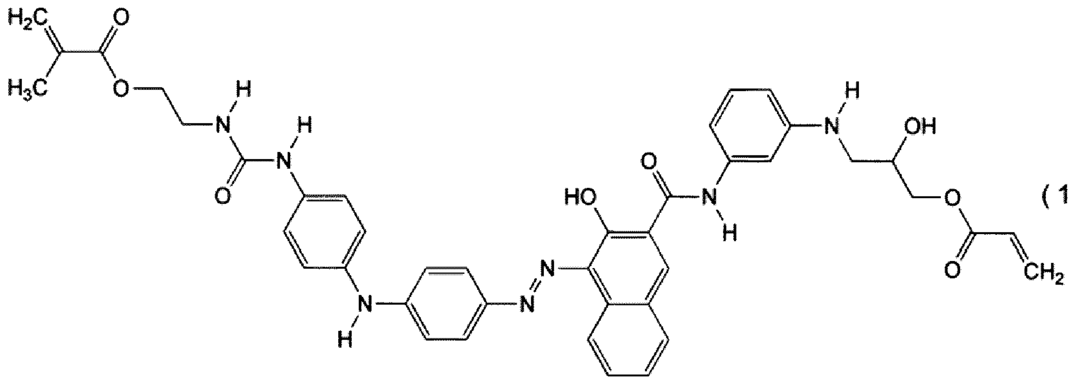

( 1 - 16 )

A reference compound (hereinafter, may be also referred to as "azo colorant 17") represented by the following formula (1-17):

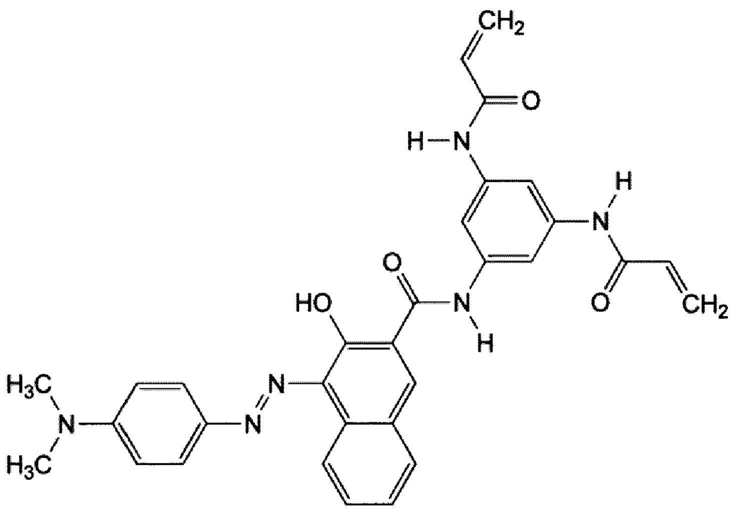

( 1 - 17 )

A reference compound (hereinafter, may be also referred to as "azo colorant 18") represented by the following formula (1-18):

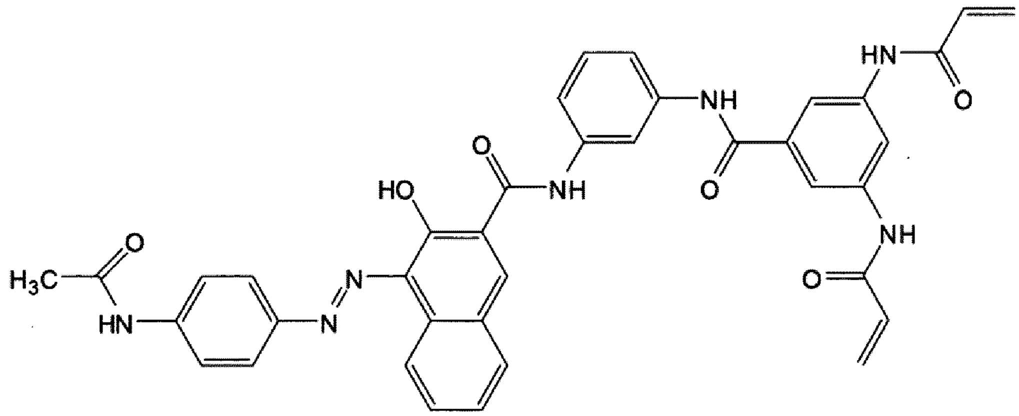

( 1 - 18 )

Production Method of Monoazo Colorant

The monoazo colorant of the embodiment of the present invention may be synthesized by a well-known method. As one example, a synthesis method of the azo colorant 1 is shown below in which a coupling agent and a diazotized base are coupled.

Synthesis of Coupling Agent

3-Hydroxy-2-naphthoic acid (3'-aminoanilide) is dispersed in THF (tetrahydrofuran), and acryloyl chloride is added dropwise while cooling therearound. After completion of the dropwise addition, the mixture is stirred at room temperature for several hours. Then an aqueous potassium carbonate solution is added dropwise thereto, and after completion of the dropwise addition, the mixture is stirred at a reflux temperature for several hours. After cooling, the mixture is filtered and the filtrate is washed with methanol and water in this order, and dried to obtain 3-hydroxy-2-naphthoic acid (3'-acryloylaminoanilide) as a coupling agent.

Synthesis of Base

2,5-Dimethoxy-4-nitroaniline is dispersed in acetone, and acryloyl chloride is added dropwise while cooling therearound. After completion of the dropwise addition, the mixture is stirred at room temperature for several hours. Then triethylamine is added dropwise thereto, and after completion of the dropwise addition, the mixture is stirred for several hours. After completion of the stirring, precipitates are filtered, washed with water, and thereafter dried to obtain N-(2,5-dimethoxy-4-nitrophenyl)acrylamide as a nitro form.

A reducing agent prepared by adding iron powder and concentrated hydrochloric acid into methanol is heated to 45°C, and then the nitro form is added thereto. After the addition, the mixture is stirred for several hours. After completion of the reaction, the mixture is neutralized by adding potassium carbonate, and subjected to hot filtration. The filtrate is concentrated in vacuo, and precipitates are washed with water and dried to obtain N-(4-amino-2,5-dimethoxyphenyl)acrylamide as a base.

Coupling

An aqueous sodium acetate trihydrate solution is added to an N,N-dimethylacetamide solution of the coupling agent. Then the base is diazotized according to a routine method, and a solution of the resultant diazo compound is added dropwise into the solution of the coupling agent to permit coupling, whereby the azo colorant 1 can be obtained.

Other monoazo colorant can be also synthesized in accordance with the method described above with appropriate alteration of the coupling agent and/or the base.

Ophthalmic Lens Material

The material for an ophthalmic lens of the embodiment of the present invention is produced from a composition containing the monoazo colorant of the embodiment of the present invention and a monomer for an ophthalmic lens having a polymerizable group, and thus contains a copolymer of the monoazo colorant and the monomer for an ophthalmic lens. The ophthalmic lens material has high solubility in the monomer for an ophthalmic lens having a polymerizable group and great color fastness with the monomer for an ophthalmic lens having a polymerizable group, whereby uniformly

coloring is permitted accompanied by superior elution resistance.

The polymerizable group included in the monomer for an ophthalmic lens is not particularly limited, and examples of the polymerizable group include a vinyl group, an allyl group, an acryl group, a methacryl group, an epoxy group, a mercapto group, a cyano group, an isocyano group, an amino group, and the like.

The monomer for an ophthalmic lens having a polymerizable group is not particularly limited, and is exemplified by a monomer having a polydimethylsiloxane structure (hereinafter, may be also referred to as "monomer A"), a monomer for an ophthalmic lens that is hydrophilic (hereinafter, may be also referred to as "monomer B"), or a nonhydrous monomer for an ophthalmic lens (hereinafter, may be also referred to as "monomer C").

The polydimethylsiloxane structure as referred to is a structure represented by the following formula (11):

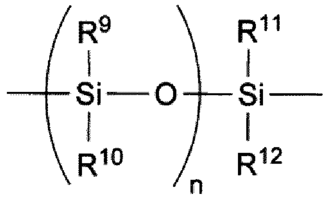

(11)

It is preferred that the monomer A having a polymerizable group (monomer having a polydimethylsiloxane structure) used as the monomer for an ophthalmic lens further has a polydimethylsiloxane structure and a polymerizable group linked via a urethane bond. Such a monomer A is a component having an elastic bond, i.e., an urethane bond, thereby imparting properties of: leading to reinforcement by way of a siloxane moiety without deteriorating flexibility and/or oxygen permeability of the material; and making the material nonfragile by imparting elastic resilience to improve mechanical strength. In addition, due to having a silicone chain in the molecular chain, the monomer A can impart high oxygen permeability to the ophthalmic lens material. Moreover, due to having a polymerizable group and being copolymerized with other copolymerizable component via such a polymerizable group, the monomer A has superior properties of imparting to the resultant ophthalmic lens material not only a physical reinforcing effect by way of entanglement of molecules, but a reinforcing effect by way of a chemical bond (i.e., covalent bond). In other words, the monomer A having a polydimethylsiloxane structure and a polymerizable group linked via a urethane bond can serve as a high-molecular crosslinkable monomer.

A typical example of the monomer A having a polydimethylsiloxane structure and a polymerizable group linked via a urethane bond is represented by the following general formula (14).

$$A^1\text{-}U^1\text{-}(S^1\text{-}U^2)_n\text{-}S^2\text{-}U^3\text{-}A^2 \qquad (14)$$

wherein, in the formula (14), $A^1$ represents a group represented by the following general formula (15):

$$Y^{11}\text{-}Z^{11}\text{-}R^{13}\text{-} \qquad (15)$$

wherein, in the formula (15), $Y^{11}$ represents a (meth)acryloyl group, a vinyl group or an allyl group; $Z^{11}$ represents an oxygen atom or a direct bond, $R^{13}$ represents a direct bond or an alkylene group having 1 to 12 carbon atoms that is linear or branched or has an aromatic ring.

$A^2$ represents a group represented by the following general formula (16):

$$\text{-}R^{14}\text{-}Z^{12}\text{-}Y^{12} \qquad (16)$$

wherein, in the formula (16), $Y^{12}$ represents a (meth)acryloyl group, a vinyl group or an allyl group; $Z^{12}$ represents an oxygen atom or a direct bond; and $R^{14}$ represents a direct bond or an alkylene group having 1 to 12 carbon atoms that is linear or branched or has an aromatic ring, wherein $Y^{11}$ in the general in the formula (15) and $Y^{12}$ in the general in the formula (16) may be identical or different.

$U^1$ represents a group represented by the following general formula (17):

$$\text{-}X^{11}\text{-}E^{11}X^{15}\text{-}R^{15}\text{-} \qquad (17)$$

wherein, in the formula (17), $X^{11}$ and $X^{15}$ are each independently selected from a direct bond, an oxygen atom and an alkylene glycol group; $E^{11}$ represents a -NHCO- group (wherein, in this case, $X^{11}$ represents a direct bond; $X^{15}$ represents an oxygen atom or an alkylene glycol group; and $E^{11}$ and $X^{15}$ taken together represent a urethane bond), a -CONH- group (wherein, in this case, $X^{11}$ represents an oxygen atom or an alkylene glycol group; $X^{15}$ represents a direct bond;

and $E^{11}$ and $X^{11}$ taken together represent a urethane bond) or a divalent group derived from a diisocyanate selected from the group consisting of saturated or unsaturated aliphatic, alicyclic and aromatic diisocyanates (wherein, in this case, $X^{11}$ and $X^{15}$ are each independently selected from an oxygen atom and an alkylene glycol group; and $E^{11}$ represents two urethane bonds formed between $X^{11}$ and $X^{15}$); and $R^{15}$ represents an alkylene group having a linear or branched chain having 1 to 6 carbon atoms.

$S^1$ and $S^2$ each independently represent a group represented by the following general formula (18):

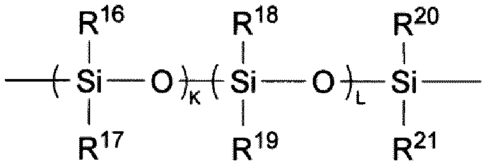 (18)

wherein, in the formula (18), $R^{16}$, $R^{17}$, $R^{18}$, $R^{19}$, $R^{20}$ and $R^{21}$ each independently represent an alkyl group having 1 to 6 carbon atoms, a fluorine-substituted alkyl group, a phenyl group or a hydrogen atom; K is an integer of 10 to 100; L is an integer of 0 or 1 to 90; and the sum of K and L is an integer of 10 to 100.

$U^2$ represents a group represented by the following general formula (19):

$$-R^{22}-X^{17}-E^{14}-X^{18}-R^{23}- \quad (19)$$

wherein, in the formula (19), $R^{22}$ and $R^{23}$ each independently represent an alkylene group having a linear or branched chain having 1 to 6 carbon atoms; $X^{17}$ and $X^{18}$ each independently represent an oxygen atom or an alkylene glycol group; $E^{14}$ represents a divalent group derived from a diisocyanate selected from the group consisting of saturated or unsaturated aliphatic, alicyclic and aromatic diisocyanates (wherein, in this case, $E^{14}$ represents two urethane bonds formed between $X^{17}$ and $X^{18}$.

$U^3$ represents a group represented by the following general formula (20):

$$-R^{24}-X^{16}-E^{12}-X^{12}- \quad (20)$$

wherein, in the formula (20), $R^{24}$ represents an alkylene group having a linear or branched chain having 1 to 6 carbon atoms; $X^{12}$ and $X^{16}$ are each independently selected from a direct bond, an oxygen atom and an alkylene glycol group, $E^{12}$ represents a -NHCO- group (wherein, in this case, $X^{12}$ represents an oxygen atom or an alkylene glycol group; $X^{16}$ represents a direct bond; and $E^{12}$ represents a urethane bond together with $X^{12}$), a -CONH- group (wherein, in this case, $X^{12}$ represents a direct bond; $X^{16}$ represents an oxygen atom or an alkylene glycol group; $E^{12}$ represents a urethane bond formed together with $X^{16}$) or a divalent group derived from a diisocyanate selected from the group consisting of saturated or unsaturated aliphatic, alicyclic and aromatic diisocyanates (wherein, in this case, $X^{12}$ and $X^{16}$ are each independently selected from an oxygen atom and an alkylene glycol group; $E^{12}$ represents two urethane bonds formed between $X^{12}$ and $X^{16}$.

"n" is an integer of 0 to 10.

In the above general formula (14), $Y^{11}$ and $Y^{12}$ both represent a polymerizable group, and in light of capability of easily copolymerizing with the monoazo colorant, a (meth)acryloyl group is particularly preferred.

In the above general formula (14), $Z^{11}$ and $Z^{12}$ both represent an oxygen atom or a direct bond, and preferably an oxygen atom. $R^{13}$ and $R^{14}$ both represent a direct bond or an alkylene group having 1 to 12 carbon atoms that is linear or branched or has an aromatic ring, and preferably an alkylene group having 2 to 4 carbon atoms. $U^1$, $U^2$ and $U^3$ represent a group that includes a urethane bond in the molecular chain.

In $U^1$ and $U^3$ in the above general formula (14), $E^{11}$ and $E^{12}$ each represent a -CONH-group, a -NHCO- group or a divalent group derived from a diisocyanate selected from the group consisting of saturated or unsaturated aliphatic, alicyclic and aromatic diisocyanates as described above. Examples of the divalent group derived from a diisocyanate selected from the group consisting of saturated or unsaturated aliphatic, alicyclic and aromatic diisocyanates include: divalent groups derived from saturated aliphatic diisocyanate such as ethylene diisocyanate 1,3-diisocyanatepropane and hexamethylene diisocyanate; divalent groups derived from alicyclic diisocyanate such as 1,2-diisocyanatecyclohexane, bis(4-isocyanatecyclohexyl)methane and isophorone diisocyanate; divalent groups derived from aromatic diisocyanate such as tolylene diisocyanate and 1,5-diisocyanatenaphthalene; and divalent groups derived from unsaturated aliphatic diisocyanate such as 2,2'-diisocyanate diethylfumarate. Among these examples, in light of availability and capability of imparting strength to lenses, a divalent group derived from hexamethylene diisocyanate, a divalent group derived from tolylene diisocyanate and a divalent group derived from isophorone diisocyanate are preferred.

In $U^2$ in the above general formula (14), $E^{14}$ represents a divalent group derived from a diisocyanate selected

from the group consisting of saturated or unsaturated aliphatic, alicyclic and aromatic diisocyanates as described above. Examples of the divalent group derived from a diisocyanate selected from the group consisting of saturated or unsaturated aliphatic, alicyclic and aromatic diisocyanates include divalent groups similar to those in connection with $U^1$ and $U^3$. Among these examples, in light of availability and capability of imparting strength to lenses, a divalent group derived from hexamethylene diisocyanate, a divalent group derived from tolylene diisocyanate and a divalent group derived from isophorone diisocyanate are preferred. In addition, $E^{14}$ represents two urethane bonds formed between $X^{17}$ and $X^{18}$. It is preferred that $X^{17}$ and $X^{18}$ each independently represent an oxygen atom or an alkylene glycol group having 1 to 6 carbon atoms, and $R^{22}$ and $R^{23}$ each independently represent an alkylene group having a linear or branched chain having 1 to 6 carbon atoms.

$X^{11}$, $X^{15}$, $X^{17}$, $X^{18}$, $X^{12}$ and $X^{16}$ preferably represent an alkylene glycol group having 1 to 20 carbon atoms. The alkylene glycol group having 1 to 20 carbon atoms is represented by the following general formula (21).

$$-O-(C_xH_{2x}-O)_y- \qquad (21)$$

wherein, x is an integer of 1 to 4, and y is an integer of 1 to 5.

Examples of the fluorine-substituted alkyl group represented by $R^{16}$, $R^{17}$, $R^{18}$, $R^{19}$, $R^{20}$ and $R^{21}$ include groups represented by: $-(CH_2)_m-C_pF_{2p+1}$ (wherein, m is an integer of 1 to 10, and p is an integer of 1 to 10). Specific examples of such a fluorine-substituted alkyl group include: linear fluorine-substituted alkyl group such as a 3,3,3-trifluoro-n-propyl group, a 2-(perfluorobutyl)ethyl group and a 2-(perfluorooctyl)ethyl group; branched fluorine-substituted alkyl groups such as a 2-(perfluoro-5-methylhexyl)ethyl group, and the like. When the amount of the compound having such a fluorine-substituted alkyl group blended is increased, resistance against lipid deposition of the resultant lens is improved.

In the above general formula (18) represented by $S^1$ and $S^2$, K is an integer of 10 to 100; L is an integer of 0 or 1 to 90; and the sum of K and L is an integer of 10 to 100 and preferably 10 to 80. When the sum of K and L is greater than 100, miscibility with the monoazo colorant, the polymeric azo colorant or the like may be inferior due to a great molecular weight, leading to phase separation in polymerization, whereby a uniform and transparent lens may not be obtained. In contrast, when the sum of K and L is less than 10, oxygen permeability of the resultant lens may be inferior, and flexibility also tends to be lowered.

In the above general formula (14), n is an integer of 0 to 10, and preferably an integer of 0 to 5. When n is greater than 10, miscibility with the other composition may be deteriorated due to a great molecular weight of the silicone compound similarly to the case described above, leading to phase separation in polymerization, whereby a uniform and transparent ophthalmic lens material may not be obtained.

The monomer A having a polydimethylsiloxane structure and a polymerizable group linked via a urethane bond may further have a hydrophilic polymer structure. This structure improves miscibility of the monomer A with the monomer B that is hydrophilic when these two monomers are used, and thus can improve wettability with water of the material for an ophthalmic lens containing these monomers. Examples of the structure of a hydrophilic polymer moiety include at least one type of polymers obtained by polymerizing a zwitterionic group-containing monomer such as polyethylene glycol, polypropylene glycol, polyvinyl alcohol, polyvinylpyrrolidone, poly(meth)acrylic acid, a poly(meth)acrylic acid salt, poly(2-hydroxyethyl (meth)acrylate), polytetrahydrofuran, polyoxetane, polyoxazoline, polydimethylacrylamide, polydiethylacrylamide and poly(2-methacryloyloxyethylphosphorylcholine). The molecular weight of this hydrophilic polymer structure moiety is 100 to 1,000,000, and preferably 1,000 to 500,000. When the molecular weight is less than 100, imparting hydrophilicity sufficient for dissolving the monomer A in the hydrophilic monomer B is likely to be impossible. On the other hand, when the molecular weight exceeds 1,000,000, a hydrophilic domain and/or a hydrophobic domain may be so large that a transparent ophthalmic lens material is less likely to be obtained.

Specific examples of the monomer A having a polydimethylsiloxane structure and a polymerizable group linked via a urethane bond include a compound represented by the following formula (22), and a compound represented by a formula (23).

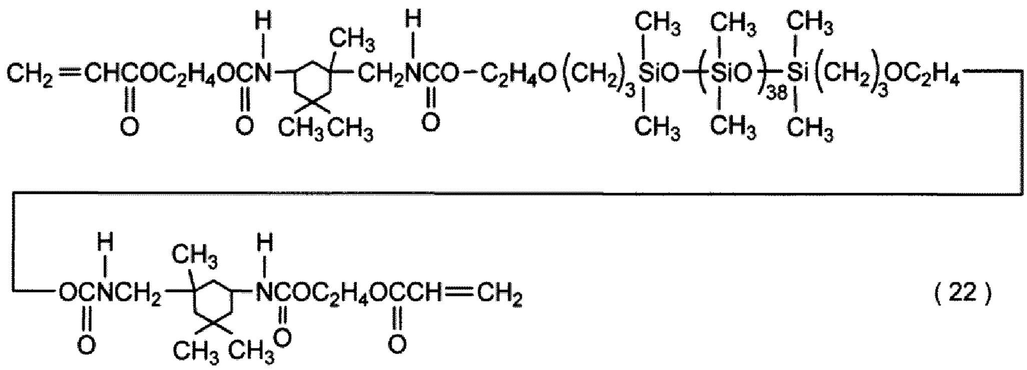

(22)

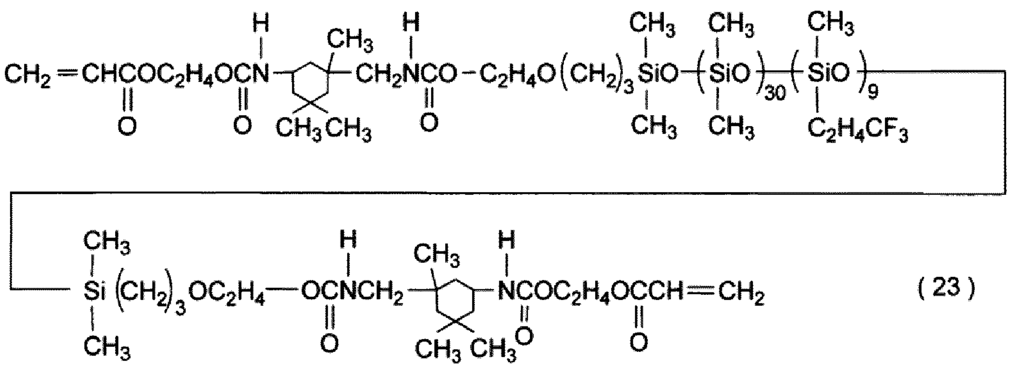

(23)

It is to be noted that in the case in which the ophthalmic lens desired is a hydrous ophthalmic lens, the monomer B as a component of the composition of the material for an ophthalmic lens may be added to the monomer A. In this case, the monomer A is included in an amount of preferably no less than 10 parts by mass and no greater than 90 parts by mass with respect to 100 parts by mass of the entire polymer components (except for a crosslinking agent, an ultraviolet ray absorbing agent, a water-soluble organic solvent and the colorant, the same applies in the following). When the amount of the monomer A included is less than the lower limit described above, sufficient oxygen permeability required for an ophthalmic lens may not be attained. To the contrary, when the amount of the monomer A included is beyond the upper limit described above, the content of the monomer B relatively decreases, whereby imparting a hydrous property sufficient for an ophthalmic lens may be difficult.

As the monomer B, for example, a hydrophilic monomer exemplified in the pamphlet of PCT International Publication No. 2005/116728 on page 15, and a pyrrolidone derivative, N-substituted acrylamide and a hydrophilic monomer exemplified in the pamphlet of PCT International Publication No. 2004/063795 on page 15, pages 20 to 21 and pages 23 to 24 may be used.

Specific examples of the monomer B include e.g., (meth)acrylamide; methoxyalkyl acrylate such as 2-methoxyethyl acrylate; hydroxyalkyl (meth)acrylate such as 2-hydroxyethyl (meth)acrylate, hydroxypropyl (meth)acrylate and hydroxybutyl (meth)acrylate; (alkyl)aminoalkyl (meth)acrylate such as 2-dimethylaminoethyl (meth)acrylate and 2-butylaminoethyl (meth)acrylate; alkylene glycol mono(meth)acrylate such as ethylene glycol mono(meth)acrylate and propylene glycol mono(meth)acrylate; polyalkylene glycol mono(meth)acrylate such as polyethylene glycol mono(meth)acrylate and polypropylene glycol mono(meth)acrylate; ethylene glycol allyl ether; ethylene glycol vinyl ether; (meth)acrylic acid; aminostyrene; hydroxystyrene; vinyl acetate; glycidyl (meth)acrylate; allyl glycidyl ether; vinyl propionate; N,N-dimethylacrylamide, N,N-dimethylmethacrylamide, N,N-diethylacrylamide, N,N-diethylmethacrylamide, N-(2-hydroxyethyl)methacrylamide, N-isopropyl (meth)acrylamide, N-(2-hydroxyethyl)acrylamide, acryloylmorpholine, methacryloylmorpholine; N-vinyl-2-pyrrolidone, N-vinyl-3-methyl-2-pyrrolidone, N-vinyl-4-methyl-2-pyrrolidone, N-vinyl-5-methyl-2-pyrrolidone, N-vinyl-3-ethyl-2-pyrrolidone, N-vinyl-4,5-dimethyl-2-pyrrolidone, N-vinyl-5,5-dimethyl-2-pyrrolidone, N-vinyl-3,3,5-trimethyl-2-pyrrolidone, N-vinyl-2-piperidone, N-vinyl-3-methyl-2-piperidone, N-vinyl-4-methyl-2-piperidone, N-vinyl-5-methyl-2-piperidone, N-vinyl-6-methyl-2-piperidone, N-vinyl-6-ethyl-2-piperidone, N-vinyl-3,5-dimethyl-2-piperidone and N-vinyl-4,4-dimethyl-2-piperidone, and N-vinyllactam such as N-vinyl-2-caprolactam, N-vinyl-3-methyl-2-caprolactam, N-vinyl-4-methyl-2-caprolactam, N-vinyl-7-methyl-2-caprolactam, N-vinyl-7-ethyl-2-caprolactam, N-vinyl-3,5-dimethyl-2-caprolactam, N-vinyl-4,6-dimethyl-2-caprolactam and N-vinyl-3,5,7-trimethyl-2-caprolactam; N-vinylamide such as N-vinylformamide, N-vinyl-N-methylformamide, N-vinyl-N-ethylformamide, N-vinylacetamide, N-vinyl-N-methylacetamide, N-vinyl-N-ethylacetamide and N-vinylphthalimide; pyrrolidone derivatives having a methylene group as a polymerizable group such as 1-methyl-3-methylene-2-pyrrolidone, 1-ethyl-3-methylene-2-pyrrolidone, 1-methyl-5-methylene-2-pyrrolidone, 1-ethyl-5-methylene-2-pyrrolidone, 5-methyl-3-methylene-2-pyrrolidone, 5-ethyl-3-methylene-2-pyrrolidone, 1-n-propyl-3-methylene-2-pyrrolidone, 1-n-propyl-5-methylene-2-pyrrolidone, 1-i-propyl-3-methylene-2-pyrrolidone, 1-i-propyl-5-methylene-2-pyrrolidone, 1-n-butyl-3-methylene-2-pyrrolidone and 1-t-butyl-3-methylene-2-pyrrolidone, and the like.

The monomer B is preferably included in an amount of no less than 10 parts by mass and no greater than 90 parts by mass with respect to 100 parts by mass of the entire polymer components. When the amount of the monomer B included is less than the lower limit described above, expected hydrophilicity may not be exhibited. To the contrary, when the amount of the monomer B included is beyond the upper limit described above, it is possible to lead to failure in exhibiting expected oxygen permeability.

Moreover, in the case in which a desired ophthalmic lens is a nonhydrous ophthalmic lens which does not substantially contain moisture, the monomer C as a component of the material for a lens may be added to the monomer A. In the case of a nonhydrous ophthalmic lens material, the monomer A is included in an amount of preferably no less than 5 parts by mass and and less than 100 parts by mass with respect to 100 parts by mass of the entire polymer components. When the amount of the monomer A included is less than the lower limit described above, sufficient oxygen permeability required for an ophthalmic lens may not be attained. Furthermore, the monomer C is included in an amount of preferably no greater than 95 parts by mass with respect to 100 parts by mass of the entire polymer components. When the amount of the monomer C included is beyond the upper limit described above, the ophthalmic lens material may be fragile.

Specific examples of the monomer C include e.g., (meth)acrylic acid esters such as alkyl (meth)acrylate, siloxanyl (meth)acrylate, fluoroalkyl (meth)acrylate, phenoxyalkyl (meth)acrylate and phenylalkyl (meth)acrylate, alkoxyalkyl (meth)acrylate, and the like.

Further, as the monomer for an ophthalmic lens having a polymerizable group, a (meth)acrylic acid derivative, a styrene derivative, an allyl group-containing compound, a vinyl group-containing compound, an exo-methylene group-containing compound, and the like are also preferred. It is to be noted that some of these monomers may be included in a group consisting of the monomer A, the monomer B and the monomer C, and each subclass thereof defined above.

Examples of the (meth)acrylic acid derivative include linear, branched or cyclic alkyl (meth)acrylate, alkoxyalkyl (meth)acrylate and alkylthioalkyl (meth)acrylate such as methyl (meth)acrylate, ethyl (meth)acrylate, isopropyl (meth)acrylate, n-propyl (meth)acrylate, isobutyl (meth)acrylate, n-butyl (meth)acrylate, 2-ethylhexyl (meth)acrylate, n-octyl (meth)acrylate, n-decyl (meth)acrylate, n-dodecyl (meth)acrylate, pentyl (meth)acrylate, n-pentyl (meth)acrylate, hexyl (meth)acrylate, heptyl (meth)acrylate, nonyl (meth)acrylate, stearyl (meth)acrylate, cyclopentyl (meth)acrylate, cyclohexyl (meth)acrylate, 2-ethoxyethyl (meth)acrylate, 3-ethoxypropyl (meth)acrylate, 2-methoxyethyl (meth)acrylate, 3-methoxypropyl (meth)acrylate, ethylthioethyl (meth)acrylate and methylthioethyl (meth)acrylate, vinyl (meth)acrylate, and the like.

Also, examples of the silicon-containing (meth)acrylic acid derivative include trimethylsiloxydimethylsilylmethyl (meth)acrylate, trimethylsiloxydimethylsilylpropyl (meth)acrylate, methylbis(trimethylsiloxy)silylpropyl (meth)acrylate, tris(trimethylsiloxy)silylpropyl (meth)acrylate, mono[methylbis(trimethylsiloxy)siloxy]bis(trimethylsiloxy)sily lpropyl (meth)acrylate, tris[methylbis(trimethylsiloxy)siloxy]silylpropyl (meth)acrylate, methylbis(trimethylsiloxy)silylpropylglyceryl (meth)acrylate, tris(trimethylsiloxy)silylpropylglyceryl (meth)acrylate, mono[methylbis(trimethylsiloxy)siloxy]bis(trimethylsiloxy)sily lpropylglyceryl (meth)acrylate, trimethylsilylethyltetramethyldisiloxypropylglyceryl (meth)acrylate, trimethylsilylmethyl (meth)acrylate, trimethylsilylpropyl (meth)acrylate, trimethylsilylpropylglyceryl (meth)acrylate, trimethylsiloxydimethylsilylpropylglyceryl (meth)acrylate, methylbis(trimethylsiloxy)silylethyltetramethyldisiloxymethyl (meth)acrylate, tetramethyltriisopropylcyclotetrasiloxanylpropyl (meth)acrylate, tetramethyltriisopropylcyclotetrasiloxybis(trimethylsiloxy)sil ylpropyl (meth)acrylate, and the like.

Examples of the styrene derivative include styrene, methylstyrene, ethylstyrene, propylstyrene, butylstyrene, t-butylstyrene, isobutylstyrene, pentylstyrene, methyl-$\alpha$-methylstyrene, ethyl-$\alpha$-methylstyrene, propyl-$\alpha$-methylstyrene, butyl-$\alpha$-methylstyrene, t-butyl-$\alpha$-methylstyrene, isobutyl-$\alpha$-methylstyrene, pentyl-$\alpha$-methylstyrene, and the like.

Furthermore, examples of silicon-containing styrene derivative include e.g., tris(trimethylsiloxy)silylstyrene, bis(trimethylsiloxy)methylsilylstyrene, (trimethylsiloxy)dimethylsilylstyrene, tris(trimethylsiloxy)siloxydimethylsilylstyrene,[bis(trimethyl siloxy)methylsiloxy]dimethylsilylstyrene, (trimethylsiloxy)dimethylsilylstyrene, heptamethyltrisiloxanylstyrene, nonamethyltetrasiloxanylstyrene, pentadecamethylheptasiloxanylstyrene, heneicosamethyldecasiloxanylstyrene, heptacosamethyltridecasiloxanylstyrene, hentriacontamethylpentadecasiloxanylstyrene, trimethylsiloxypentamethyldisiloxymethylsilylstyrene, tris(pentamethyldisiloxy)silylstyrene, tris(trimethylsiloxy)siloxybis(trimethylsiloxy)silylstyrene, bis(heptamethyltrisiloxy)methylsilylstyrene, tris[methylbis(trimethylsiloxy)siloxy]silylstyrene, trimethylsiloxybis[tris(trimethylsiloxy)siloxy]silylstyrene, heptakis(trimethylsiloxy)trisilylstyrene, nonamethyltetrasiloxyundecylmethylpentasiloxymethylsilylstyren e, tris[tris(trimethylsiloxy)siloxy]silylstyrene, (tristrimethylsiloxyhexamethyl)tetrasiloxy[tris(trimethylsilox y)siloxy]trimethylsiloxysilylstyrene, nonakis(trimethylsiloxy)tetrasilylstyrene, bis(tridecamethylhexasiloxy)methylsilylstyrene, heptamethylcyclotetrasiloxanylstyrene, heptamethylcyclotetrasiloxybis(trimethylsiloxy)silylstyrene, tripropyltetramethylcyclotetrasiloxanylstyrene, trimethylsilylstyrene, and the like.

Examples of the allyl group-containing compound include ethylene glycol diallyl carbonate, trimellitic acid triallyl ester, triallyl isocyanurate, and the like.

Examples of the vinyl group-containing compound include vinyl (meth)acrylate, divinylbenzene, ethylene glycol di(meth)acrylate, trimethylolpropane tri(meth)acrylate, urethane modified (meth)acrylate, epoxy modified (meth)acrylate and polyester modified (meth)acrylate containing at least two or more (meth)acryloxy groups in one molecule, and the like.

Examples of the exomethylene group-containing compound include methylenecyclohexane, 5-methylenecyclopentadiene, dimethyl 3-methylenecyclopropane-1,2-dicarboxylate, $\alpha$-methylene-$\gamma$-butyrolactone, and the like.

Also, the monoazo colorant is included in an amount of preferably no less than 0.001 parts by mass and no greater than 0.5 parts by mass with respect to 100 parts by mass of the entire polymer components. When the amount of the monoazo colorant included is less than 0.001 parts by mass, the coloring effect may not be sufficient achieved. Whereas, when the amount of the monoazo colorant included is greater than 0.5 parts by mass, the color tone may be so dark leading to loss of practicability, or it may be difficult to transmit the visible light ray.

It is to be noted that the composition may contain other component such as a crosslinking agent, in addition to the monoazo colorant of the embodiment of the present invention and the monomer for an ophthalmic lens having a polymerizable group. The crosslinking agent can adjust the crosslinking density, flexibility, and/or hardness of the ophthalmic lens material obtained.

Examples of the crosslinking agent include e.g., allyl methacrylate, ethylene glycol di(meth)acrylate, diethylene glycol di(meth)acrylate, vinyl methacrylate, 4-vinylbenzyl methacrylate, 3-vinylbenzyl methacrylate, methacryloyloxyethyl acrylate, ethylene glycol dimethacrylate, diethylene glycol dimethacrylate, diethylene glycol diallyl ether, triethylene glycol dimethacrylate, tetraethylene glycol dimethacrylate, propylene glycol dimethacrylate, dipropylene glycol dimethacrylate, butanediol dimethacrylate, trimethylolpropane trimethacrylate, 2,2-bis(p-methacryloyloxyphenyl)hexafluoropropane, 2,2-bis(m-methacryloyloxyphenyl)hexafluoropropane, 2,2-bis(o-methacryloyloxyphenyl)hexafluoropropane, 2,2-bis(p-methacryloyloxyphenyl)propane, 2,2-bis(m-methacryloyloxyphenyl)propane, 2,2-bis(o-methacryloyloxyphenyl)propane, 1,4-bis(2-methacryloyloxyhexafluoroisopropyl)benzene, 1,3-bis(2-methacryloyloxyhexafluoroisopropyl)benzene, 1,2-bis(2-methacryloyloxyhexafluoroisopropyl)benzene, 1,4-bis(2-methacryloyloxyisopropyl)benzene, 1,3-bis(2-methacryloyloxyisopropyl)benzene, 1,2-bis(2-methacryloyloxyisopropyl)benzene, and the like. These crosslinking agents may be used either alone, or as a mixture of two or more thereof.

The proportion of the crosslinking agent used is preferably no less than 0.05 parts by mass and no greater than 1 part by mass, and more preferably no less than 0.1 parts by mass and no greater than 0.8 parts by mass with respect to 100 parts by mass of the entire polymer components. When the proportion of the crosslinking agent used falls within the above range, both mechanical strength and flexibility can be achieved.

Method for Producing Ophthalmic Lens Material

The ophthalmic lens material can be produced with a method of polymerization and molding of an ophthalmic lens material commonly performed by persons skilled in the art. For example, 1) a method which includes allowing for polymerization in an appropriate mold or vessel in which a composition for an ophthalmic lens material was mixed to obtain a material (polymer) having a rod shape, block shape or plate shape, followed by processing to give a desired shape by mechanical processing such as cutting processing or grinding processing, 2) a method which includes providing a mold corresponding to a desired shape, allowing polymerization of a composition for an ophthalmic lens material in the mold to obtain a cured matter, and subjecting to finish processing mechanically as needed, and the like may be employed. Of these production methods, the following specific method is preferred.

More specifically, the ophthalmic lens material of the embodiment of the present invention may be produced by a method including the steps of:

(A) preparing a composition for an ophthalmic lens material containing the monoazo colorant, and a monomer for an ophthalmic lens having a polymerizable group;
(B) transferring the composition into a mold;
(C) curing the composition in the mold by heating, irradiation with an ultraviolet ray, a visible ray or a combination of an ultraviolet ray and a visible ray, or both the heating and the irradiation to obtain a cured matter; and
(D) releasing the cured matter.

Furthermore, in the case in which the ophthalmic lens material is a hydrous ophthalmic lens material, the material may be produced by a method further including the steps of:

(E) hydrating the cured matter; and
(F) removing an unreacted material from the cured matter hydrated.

In the embodiment of the present invention, the composition preferably contains a water soluble organic solvent in order to improve homogeneity of the components in the composition. When the water soluble organic solvent is included in a slight amount with respect to the polymerizable component, unreacted monomers can be diffused in the system also after the polymerization reaction proceeds, thereby enabling them to participate in the polymerization reaction. In other words, the water soluble organic solvent can decrease residual polymerizable components. Furthermore, since the organic solvent used is water soluble, it can be readily replaced with water in the step of an elution treatment carried out later.

As the water soluble organic solvent, an alcohol having 1 to 4 carbon atoms such as methanol, ethanol, 1-propanol or 2-propanol, acetone, methyl ethyl ketone, dimethyl formamide, dimethyl sulfoxide, acetonitrile, N-methyl-2-pyrrolidone or the like may be used. The water soluble organic solvent may be used through selecting ad libitum one which can dissolve the polymerizable component employed, depending on the type of the polymerizable component. These may be used either alone, or as a mixture of two or more thereof.

The amount of the water soluble organic solvent used is preferably no less than 0.1 parts by mass and 5 parts by mass, and more preferably no less than 0.2 parts by mass and no greater than 4 parts by mass with respect to 100 parts by mass of the entire polymer components. When the amount of the water soluble organic solvent used is below the range described above, the amount of remaining component in polymerization tends to be increased. To the contrary, when the amount of the water soluble organic solvent used is beyond the range described above, the solvent may not be completely removed in the step of the elution treatment carried out later.

The composition may contain a polymerization initiator that serves by way of heat or electromagnetic wave (i.e., ultraviolet rays, visible light rays, etc.).

Specific examples of the radical polymerization initiator that serves by way of heat include e.g., azo type polymerization initiators such as 2,2'-azobisisobutylnitrile and 2,2'-azobisdimethylvaleronitrile; peroxide type polymerization initiators such as benzoyl peroxide, tert-butyl hydroperoxide and cumene hydroperoxide, and the like.

Examples of the polymerization initiator that serves by way of electromagnetic wave include phosphine oxide type polymerization initiators such as 2,4,6-trimethylbenzoyl-diphenylphosphine oxide (TPO) and bis(2,4,6-trimethylbenzoyl)-phenylphosphine oxide; benzoin type polymerization initiators such as methylorthobenzoyl benzoate, methylbenzoyl formate, benzoin methyl ether, benzoin ethyl ether, benzoin isopropyl ether, benzoin isobutyl ether and benzoin n-butyl ether; phenone type polymerization initiators such as 2-hydroxy-2-methyl-1-phenylpropan-1-one, p-isopropyl-$\alpha$-hydroxyisobutylphenone, p-t-butyltrichloroacetophenone, 2,2-dimethoxy-2-phenylacetophenone, $\alpha$,$\alpha$-dichloro-4-phenoxyacetophenone and N,N-tetraethyl-4,4-diaminobenzophenone; 1-hydroxycyclohexyl phenyl ketone; 1-phenyl-1,2-propanedione-2-(o-ethoxycarbonyl)oxime; thioxanthone type polymerization initiators such as 2-chlorothioxanthone and 2-methylthioxanthone; dibenzosuberone; 2-ethylanthraquinone; benzophenone acrylate; benzophenone; benzyl, and the like. These may be used either alone, or as a mixture of two or more thereof.

As the material of the mold used in polymerization by way of irradiation with an an ultraviolet ray, a visible ray or a combination thereof, a commodity resin which can transmit the ultraviolet ray, the visible ray or a combination thereof required for curing of the lens material such as, for example, polypropylene, polystyrene, nylon or polyester is preferred, or alternatively, glass may be also used.

Preferable illuminance of the ultraviolet ray, the visible ray or a combination thereof when the polymer components in the mold are irradiated with an ultraviolet ray, a visible ray or a combination thereof is no less than 0.1 $mW/cm^2$ for sufficiently curing the lens material, and is preferably no greater than 50 $mW/cm^2$ for the purpose of preventing the lens material from deterioration. Also, the irradiation time is preferably 1 min or longer for sufficiently curing the lens material. The irradiation with an ultraviolet ray, a visible ray or a combination thereof may be carried out in a single step, or ultraviolet ray, visible rays or combinations thereof having different illuminance may be irradiated stepwise. In addition, during the polymerization, heating may be carried out concomitantly with or separately from irradiation with an ultraviolet ray, a visible ray or a combination thereof, whereby the polymerization reaction is accelerated, and thus effective molding of the ophthalmic lens is enabled.

In the case in which curing is executed by heating, the heating temperature is, in light of acceleration of the reaction, preferably no less than 25°C, more preferably no less than 30°C, whereas in light of suppression of deformation of the mold, preferably no greater than 100°C, and more preferably no greater than 90°C.

After completing the polymerization, cured matter obtained is subjected to mechanical processing such as cutting processing or grinding processing as needed. It is to be noted that the cutting may be carried out over entirety of at least one face or two both faces of the cured matter (copolymer), or on a part of at least one face or two both faces of the cured matter (copolymer). The ophthalmic lens material of the present invention is particularly preferably a cured matter (copolymer) subjected to cutting on at least one face or a part thereof, taking into consideration expansion of applications of use of the product such as a special-purpose lens.

It is to be noted that the step (D) is preferably carried out while the cured matter is in a dry state without using a medium such as water. According to the method for the production, since a solvent or the like is not used, a step of operation such as removal of the solvent from the cured matter which had been released from the mold is not needed;

therefore, simplification of the production step can be realized.

Furthermore, it is preferred that the step (E) of hydrating the cured matter, and the step (F) of removing an unreacted material from the cured matter hydrated be concomitantly carried out by immersing the cured matter obtained via the step (D) in any one of water, a physiological saline solution, a phosphoric acid buffer and a boric acid buffer. According to the method for the production, two steps can be concomitantly carried out; therefore, simplification of the production step can be realized.

Moreover, according to the method for producing an ophthalmic lens material, by the step (G) of subjecting the cured matter after the releasing step to a surface treatment, hydrophilicity and stain resistance can be imparted to the lens surface. As the surface treatment, for example, a dry process by way of: a step of exposing the cured matter in a plasma discharge atmosphere (i.e., a plasma treatment carried out using various types of reaction gas or inert gas, etc.); a step of irradiation of the cured matter with an ultraviolet ray or a radioactive ray; or the like may be employed. Alternatively, a step of graft polymerization on the surface of the cured matter, for example, a wet process can be also employed such as a multilayer lamination method (layer-by-layer method) carried out using polyelectrolyte, a plasma treatment or the like to produce a radical active species on the surface, followed by bringing into contact with a hydrophilic monomer to permit graft polymerization, or the like.

Ophthalmic Lens

Since the colorant contained is strongly bound to other ophthalmic lens components in the ophthalmic lens material of the embodiment of the present invention obtained in this manner, the colorant is less likely to be eluted, and thus superior elution resistance is provided. Therefore, the ophthalmic lens material can be suitably used for ophthalmic lenses such as, e.g., contact lenses, intraocular lenses, artificial cornea, cornea onlay, and cornea inlay after subjecting to other processing as needed.

EXAMPLES

Hereinafter, the present invention will be explained in more detail by way of Examples, but the present invention is not limited to the following Examples. It is to be noted that the term "part" herein is on mass basis.

Example 1: Synthesis of Azo Colorant 1 (compound represented by the above formula (1-1))

Synthesis of Coupling Agent

3-Hydroxy-2-naphthoic acid (3'-aminoanilide) in an amount of 13.92 parts was dispersed in THF (tetrahydrofuran), and thereto were gradually added 5.43 parts of acryloyl chloride dropwise while cooling therearound. After completion of the dropwise addition, the mixture was stirred at room temperature for several hours. Next, a solution prepared by dissolving 4.45 parts of potassium carbonate in 30 parts of water was added dropwise, and after completion of the dropwise addition, the mixture was stirred at reflux temperature for several hours. After cooling, the mixture was filtered, and the filtration residue was washed with methanol and water in this order, followed by drying to obtain 11.3 parts of 3-hydroxy-2-naphthoic acid (3'-acryloylaminoanilide) (yield: 75%).

melting point: 252°C
elemental analysis values (values in parentheses indicating a theoretical value): C; 72.4% (72.8%), H; 5.27% (5.24%), N; 8.11% (8.09%)

Synthesis of Base

Synthesis of Nitro Form

2,5-Dimethoxy-4-nitroaniline in an amount of 15.8 parts was dispersed in 200 parts of acetone, and thereto were gradually added 8.18 parts of acryloyl chloride while cooling therearound. After completion of the dropwise addition, the mixture was stirred at room temperature for several hours. Then 8.8 parts of triethylamine were added dropwise, and after completion of the dropwise addition, the mixture was stirred for several hours. After completion of the stirring, the solution was poured into 1,600 parts of water, and the precipitates were filtered and washed with water, followed by drying to obtain 18.9 parts of N-(2,5-dimethoxy-4-nitrophenyl)acrylamide.
melting point: 146°C

Synthesis of Base

Into 50 parts of methanol were added 5.87 parts of iron powder and 0.3 parts of concentrated hydrochloric acid as a reducing agent. This reaction solution was heated to 45°C, and then 7.57 parts of the nitro form obtained by the method described above were added over 15 min. After completion of the addition, the mixture was stirred at reflux temperature for several hours. After completion of the reaction, the solution was neutralized by adding 0.8 parts of potassium carbonate, and subjected to hot filtration. The filtrate was concentrated in vacuo, and the precipitates were washed with water, followed by drying to obtain 5.0 parts of N-(4-amino-2,5-dimethoxyphenyl)acrylamide.
melting point: 154°C

Synthesis of Azo Colorant

N-(4-amino-2,5-dimethoxyphenyl)acrylamide in an amount of 9.54 parts as a base obtained by the method described above was dissolved in 500 parts of N,N-dimethylacetamide, and thereto was added a solution prepared by dissolving 13.38 parts of sodium acetate trihydrate in 50 parts of water. Next, 6.08 parts of 3-hydroxy-2-naphthoic acid (3'-acryloylaminoanilide) as a coupling agent obtained by the method described above was diazotization according to a routine method, and the resultant diazo liquid was added dropwise to the solution of the coupling agent to permit coupling. After the mixture was stirred at room temperature for several hours and filtered, the filtration residue was sufficiently washed with 1-butanone and then with water, followed by drying to obtain 14.8 parts of the azo colorant 1 (yield: 93%). The azo colorant 1 thus obtained had a bright purple color.

melting point: 251°C
elemental analysis values (values in parentheses indicating a theoretical value): C; 69.0% (69.4%), H; 5.39% (5.36%), N; 10.68% (10.65%)
An infrared absorption spectrum of the resulting azo colorant 1 is shown in Fig. 1, and a $^1$H-NMR spectrum (standard: TMS; solvent: $CDCl_3$) is shown in Fig. 2.

Example 2: Synthesis of Azo Colorant 2 (compound represented by the above formula (1-2))

The azo colorant 2 in an amount of 17.72 parts was obtained (yield: 97%) by carrying out a reaction and a post-treatment in a similar manner to Example 1 except that 6.08 parts of N-(4-amino-2,5-dimethoxyphenyl)acrylamide in Example 1 were changed to 8.90 parts of 4-acrylamide-N-(4-amino-2,5-dimethoxyphenyl)benzamide. The azo colorant 2 thus obtained had a bright purple color. An infrared absorption spectrum of the resulting azo colorant 2 is shown in Fig. 3, and a $^1$H-NMR spectrum (standard: TMS; solvent: $CDCl_3$) thereof is shown in Fig. 4.

Reference Example 3: Synthesis of reference Azo Colorant 3 (compound represented by the above formula (1-3))

The azo colorant 3 in an amount of 19.85 parts was obtained (yield: 86%) by carrying out a reaction and a post-treatment in a similar manner to Example 1 except that 6.08 parts of N-(4-amino-2,5-dimethoxyphenyl)acrylamide in Example 1 were changed to 8.98 parts of N-(4-amino-2,5-dimethoxyphenyl)-4-tert-butylbenzamide, and that 9.54 parts of 3-hydroxy-2-naphthoic acid (3'-acryloylaminoanilide) were changed to 14.48 parts of N,N'-(5-(3-(3-hydroxy-2-naphthamide)phenylcarbamoyl)-1,3-phenylene)diacrylamide. The azo colorant 3 thus obtained had a bright purple color. An infrared absorption spectrum of the resulting azo colorant 3 is shown in Fig. 5, and a $^1$H-NMR spectrum (standard: TMS; solvent: $CDCl_3$) thereof is shown in Fig. 6.

Example 4: Synthesis of Azo Colorant 4 (compound represented by the above formula (1-4))

The azo colorant 4 in an amount of 20.94 parts was obtained (yield: 91%) by carrying out a reaction and a post-treatment in a similar manner to Example 1 except that 6.08 parts of N-(4-amino-2,5-dimethoxyphenyl)acrylamide in Example 1 were changed to 8.90 parts of 4-acrylamide-N-(4-amino-2,5-dimethoxyphenyl)benzamide, and that 9.54 parts of 3-hydroxy-2-naphthoic acid (3'-acryloylaminoanilide) were changed to 14.48 parts of N,N'-(5-(3-(3-hydroxy-2-naphthamide)phenylcarbamoyl)-1,3-phenylene)diacrylamide. The azo colorant 4 thus obtained had a bright purple color.

Example 5: Synthesis of Azo Colorant 5 (compound represented by the above formula (1-5))

The azo colorant 5 in an amount of 19.76 parts was obtained (yield: 78%) by carrying out a reaction and a post-treatment in a similar manner to Example 1 except that 6.08 parts of N-(4-amino-2,5-dimethoxyphenyl)acrylamide in Example 1 were changed to 11.23 parts of N,N'-(5-(4-amino-2,5-dimethoxyphenylcarbamoyl)-1,3-phenylene)diacrylamide, and that 9.54 parts of 3-hydroxy-2-naphthoic acid (3'-acryloylaminoanilide) were changed to 14.48 parts of N,N'-(5-(3-(3-hydroxy-2-naphthamide)phenylcarbamoyl)-1,3-phenylene)diacrylamide. The azo colorant 5 thus obtained had a bright purple color.

Reference Example 6: Synthesis of Azo Colorant 6 (compound represented by the above formula (1-6))

The reference azo colorant 6 in an amount of 18.66 parts was obtained (yield: 77%) by carrying out a reaction and a post-treatment in a similar manner to Example 1 except that 6.08 parts of N-(4-amino-2,5-dimethoxyphenyl)acrylamide in Example 1 were changed to 11.23 parts of N,N'-(5-(4-amino-2,5-dimethoxyphenylcarbamoyl)-1,3-phenylene)diacrylamide, and that 9.54 parts of 3-hydroxy-2-naphthoic acid (3'-acryloylaminoanilide) were changed to 13.33 parts of 3-hydroxy-N-(3-(3-acrylamide-4-methylbenzamide))phenyl-2-naphthamide. The azo colorant 6 thus obtained had a bright purple color.

Example 7: Synthesis of Azo Colorant 7 (compound represented by the above formula (1-7))

The azo colorant 7 in an amount of 12.18 parts was obtained (yield: 79%) by carrying out a reaction and a post-treatment in a similar manner to Example 1 except that 6.08 parts of N-(4-amino-2,5-dimethoxyphenyl)acrylamide in Example 1 were changed to 5.64 parts of N-(4-amino-2-methyl-5-methoxyphenyl)acrylamide, and that 9.54 parts of 3-hydroxy-2-naphthoic acid (3'-acryloylaminoanilide) were changed to 9.94 parts of 3-hydroxy-N-(3-methacrylamidephenyl)-2-naphthamide. The azo colorant 7 thus obtained had a bright reddish purple color.

Reference Example 8: Synthesis of Azo Colorant 8 (compound represented by the above formula (1-8))

The reference azo colorant 8 in an amount of 18.68 parts was obtained (yield: 84%) by carrying out a reaction and a post-treatment in a similar manner to Example 1 except that 6.08 parts of N-(4-amino-2,5-dimethoxyphenyl)acrylamide in Example 1 were changed to 3.73 parts of N,N-dimethyl-p-phenylenediamine, and that 9.54 parts of 3-hydroxy-2-naphthoic acid (3'-acryloylaminoanilide) were changed to 19.11 parts of N,N'-(5-(5-(2-hydroxy-11H-benzo[a]carbazole-3-carboxamide)-2-methoxyphenylcarbamoyl)-1,3-phenylene)bismethacrylamide. The azo colorant 8 thus obtained had a greenish deep blue color.

Reference Example 9: Synthesis of Azo Colorant 9 (compound represented by the above formula (1-9))

The reference azo colorant 9 in an amount of 17.81 parts was obtained (yield: 93%) by carrying out a reaction and a post-treatment in a similar manner to Example 1 except that 6.08 parts of N-(4-amino-2,5-dimethoxyphenyl)acrylamide in Example 1 were changed to 5.04 parts of 4-aminodiphenylamine, and that 9.54 parts of 3-hydroxy-2-naphthoic acid (3'-acryloylaminoanilide) were changed to 14.48 parts of N,N'-(5-(3-(3-hydroxy-2-naphthamide)phenylcarbamoyl)-1,3-phenylene)diacrylamide. The azo colorant 9 thus obtained had a bright blue color.

An infrared absorption spectrum of the resulting azo colorant 9 thus is shown in Fig. 7.

Reference Example 10: Synthesis of Azo Colorant 10 (compound represented by the above formula (1-10))

The reference azo colorant 10 in an amount of 23.44 parts was obtained (yield: 90%) by carrying out a reaction and a post-treatment in a similar manner to Example 1 except that 6.08 parts of N-(4-amino-2,5-dimethoxyphenyl)acrylamide in Example 1 were changed to 11.23 parts of N,N'-(5-(4-amino-2,5-dimethoxyphenylcarbamoyl)-1,3-phenylene)diacrylamide, and that 9.54 parts of 3-hydroxy-2-naphthoic acid (3'-acryloylaminoanilide) were changed to 15.23 parts of N-(3-(3-acrylamide-4-methoxybenzamide)phenyl)-3-hydroxyanthracene-2-carboxamide. The azo colorant 10 thus obtained had a green color.

Example 11: Synthesis of Azo Colorant 11 (compound represented by the above formula (1-11))

The azo colorant 11 in an amount of 19.37 parts was obtained (yield: 87%) by carrying out a reaction and a post-treatment in a similar manner to Example 1 except that 6.08 parts of N-(4-amino-2,5-dimethoxyphenyl)acrylamide in Example 1 were changed to 10.11 parts of 4-acrylamide-N-(4-amino-2,5-dimethoxyphenyl)benzamide, and that 9.54 parts of 3-hydroxy-2-naphthoic acid (3'-acryloylaminoanilide) were changed to 12.44 parts of 2-(3-(3-(3-hydroxy-2-naphthamide)phenyl)ureide)ethyl methacrylate. The azo colorant 11 thus obtained had a bright purple color.

Reference Example 12: Synthesis of Azo Colorant 12 (compound represented by the above formula (1-12))

The reference azo colorant 12 in an amount of 23.69 parts was obtained (yield: 93%) by carrying out a reaction and a post-treatment in a similar manner to Example 1 except that 6.08 parts of N-(4-amino-2,5-dimethoxyphenyl)acrylamide in Example 1 were changed to 3.73 parts of N,N-dimethyl-p-phenylenediamine, and that 9.54 parts of 3-hydroxy-2-naphthoic acid (3'-acryloylaminoanilide) were changed to 22.49 parts of 2,2'-(5-(3-(2-hydroxy-11H-benzo[a]carbazole-3-carboxamide)phenylcarbamoyl)-1,3-phenylene)bis(ureideethylacrylate). The azo colorant 12 thus obtained had a deep blue color.

Reference Example 13: Synthesis of Azo Colorant 13 (compound represented by the above formula (1-13))

The reference azo colorant 13 in an amount of 22.07 parts was obtained (yield: 79%) by carrying out a reaction and a post-treatment in a similar manner to Example 1 except that 6.08 parts of N-(4-amino-2,5-dimethoxyphenyl)acrylamide in Example 1 were changed to 12.13 parts of 2-(3-(4-(4-amino-2-methyl-5-methoxybenzamide)ureide)ethyl methacrylate, and that 9.54 parts of 3-hydroxy-2-naphthoic acid (3'-acryloylaminoanilide) were changed to 16.26 parts of 2-(3-(5-(3-(3-hydroxy-2-naphthamide)phenylcarbamoyl)-2-methylphenyl)ureide)ethyl methacrylate. The azo colorant 13 thus obtained had a bright reddish purple color.

Reference Example 14: Synthesis of Azo Colorant 14 (compound represented by the above formula (1-14))

The reference azo colorant 14 in an amount of 15.59 parts was obtained (yield: 89%) by carrying out a reaction and a post-treatment in a similar manner to Example 1 except that 6.08 parts of N-(4-amino-2,5-dimethoxyphenyl)acrylamide in Example 1 were changed to 5.71 parts of N-(4-amino-3-chlorophenyl)acrylamide, and that 9.54 parts of 3-hydroxy-2-naphthoic acid (3'-acryloylaminoanilide) were changed to 12.07 parts of 2-hydroxy-3-(4-(3-hydroxy-2-naphthamide)phenylamino)propyl methacrylate. The azo colorant 14 thus obtained had a bright red color.

Reference Example 15: Synthesis of Azo Colorant 15 (compound represented by the above formula (1-15))

The reference azo colorant 15 in an amount of 16.05 parts was obtained (yield: 90%) by carrying out a reaction and a post-treatment in a similar manner to Example 1 except that 6.08 parts of N-(4-amino-2,5-dimethoxyphenyl)acrylamide in Example 1 were changed to 3.73 parts of N,N-dimethyl-p-phenylenediamine, and that 9.54 parts of 3-hydroxy-2-naphthoic acid (3'-acryloylaminoanilide) were changed to 14.48 parts of N,N'-(5-(3-(3-hydroxy-2-naphthamide)phenylcarbamoyl)-1,3-phenylene)diacrylamide. The azo colorant 15 thus obtained had a deep blue color.

An infrared absorption spectrum of the resulting azo colorant 15 is shown in Fig. 8.

Reference Example 16: Synthesis of Azo Colorant 16 (compound represented by the above formula (1-16))

The reference azo colorant 16 in an amount of 17.95 parts was obtained (yield: 85%) by carrying out a reaction and a post-treatment in a similar manner to Example 1 except that 6.08 parts of N-(4-amino-2,5-dimethoxyphenyl)acrylamide in Example 1 were changed to 9.70 parts of 2-(3-(4-(4-aminophenylamino)phenyl)ureide)ethyl methacrylate, and that 9.54 parts of 3-hydroxy-2-naphthoic acid (3'-acryloylaminoanilide) were changed to 11.67 parts of 2-hydroxy-3-(3-(3-hydroxy-2-naphthamide)phenylaminopropyl acrylate. The azo colorant 16 thus obtained had a bright bluish purple color.

Reference Example 17: Synthesis of Azo Colorant 17 (compound represented by the above formula (1-17))

The reference azo colorant 17 in an amount of 14.11 parts was obtained (yield: 94%) by carrying out a reaction and a post-treatment in a similar manner to Example 1 except that 6.08 parts of N-(4-amino-2,5-dimethoxyphenyl)acrylamide in Example 1 were changed to 3.73 parts of N,N-dimethyl-p-phenylenediamine, and that 9.54 parts of 3-hydroxy-2-naphthoic acid (3'-acryloylaminoanilide) were changed to 11.53 parts of N,N'-(5-(3-hydroxy-2-naphthamide)-1,3-phenylene)diacrylamide. The azo colorant 17 thus obtained had a deep blue color.

Reference Example 18: Synthesis of Azo Colorant 18 (compound represented by the above formula (1-18))

The reference azo colorant 18 in an amount of 15.9 parts was obtained (yield: 85%) by carrying out a reaction and a post-treatment in a similar manner to Example 1 except that 6.08 parts of N-(4-amino-2,5-dimethoxyphenyl)acrylamide in Example 1 were changed to 4.11 parts of 4-aminoacetanilide, and that 9.54 parts of 3-hydroxy-2-naphthoic acid (3'-acryloylaminoanilide) were changed to 14.26 parts of N,N'-(5-(3-(3-hydroxy-2-naphthamide)phenylcarbamoyl)-1,3-phenylene)diacrylamide. The azo colorant 18 thus obtained had a reddish purple color.

An infrared absorption spectrum of the resulting azo colorant 18 is shown in Fig. 9, and a $^1$H-NMR spectrum (standard: TMS; solvent: $CDCl_3$) thereof is shown in Fig. 10.

Each hue, maximum absorption wavelength, melting point and yield of the azo colorants 1 to 18 obtained in Examples 1, 2, 4, 5, 7 and 11 and Reference Examples 3, 6, 8, 9, 10, and 12 to 18 are shown in Table 1 below.

[Table 1]

| Azo colorant | Hue | Maximum absorption wavelength (nm) | Melting point (decomposition point) (°C) | Yield (%) |
|---|---|---|---|---|
| 1 | purple | 535 | 251 | 93 |
| 2 | purple | 535 | 267 | 97 |
| 3 | purple | 535 | 271 | 86 |
| 4 | purple | 536 | 260 | 91 |
| 5 | purple | 535 | 325 | 78 |
| 6 | purple | 534 | 310 | 77 |
| 7 | reddish purple | 520 | 263 | 79 |
| 8 | greenish deep blue | 670 | 284 | 84 |
| 9 | blue | 610 | 279 | 93 |
| 10 | green | 680 | 266 | 90 |
| 11 | purple | 540 | 259 | 87 |
| 12 | deep blue | 537 | 288 | 93 |
| 13 | reddish purple | 518 | 240 | 79 |
| 14 | red | 500 | 335 | 89 |
| 15 | deep blue | 665 | 306 | 90 |
| 16 | bluish purple | 590 | 275 | 85 |
| 17 | deep blue | 665 | 291 | 94 |
| 18 | reddish purple | 520 | 256 | 85 |

The term "parts by mass" as referred to in the following description means a mass with respect to 100 parts of the total amount of polymerizable components excluding the crosslinking agent, ultraviolet ray absorbing agent, water soluble organic solvent, polymerization initiator and colorant. It is to be noted that abbreviations are as described in the following.

Monomer for Ophthalmic Lens

- macromonomer (A1): the compound represented by the above formula (22), which is obtained in accordance with a production method disclosed in the pamphlet of PCT International Publication No. 2004/063795 on pages 48 to 49.
- TRIS: tris(trimethylsiloxy)silylpropyl methacrylate
- 2-MTA: 2-methoxyethyl acrylate
- N-VP: N-vinylpyrrolidone
- DMAA: N,N-dimethylacrylamide
- 1,3-MMP: 1-methyl-3-methylene-2-pyrrolidone

Crosslinking Agent

- AMA: allyl methacrylate
- EDMA: ethylene glycol dimethacrylate

Ultraviolet Ray Absorbing Agent

- HMEPBT: 2-(2'-hydroxy-5'-methacryloyloxyethylphenyl)-2H-benzotriazole

Water Soluble Organic Solvent

- n-PrOH: 1-propanol

Polymerization Initiator

- HMPPO: 2-hydroxy-2-methyl-1-phenylpropan-1-one
- TPO: 2,4,6-trimethylbenzoyl-diphenylphosphine oxide Colorant for Comparative Examples
- azo colorant A: a compound represented by the following formula (A):

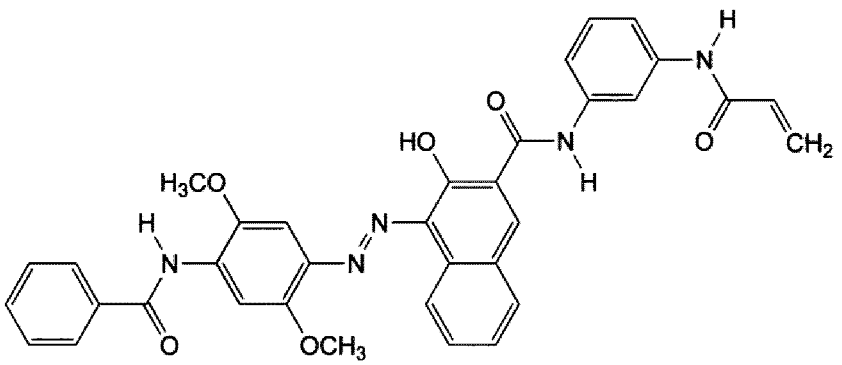

(A)

- azo colorant B: a compound represented by the following formula (B):

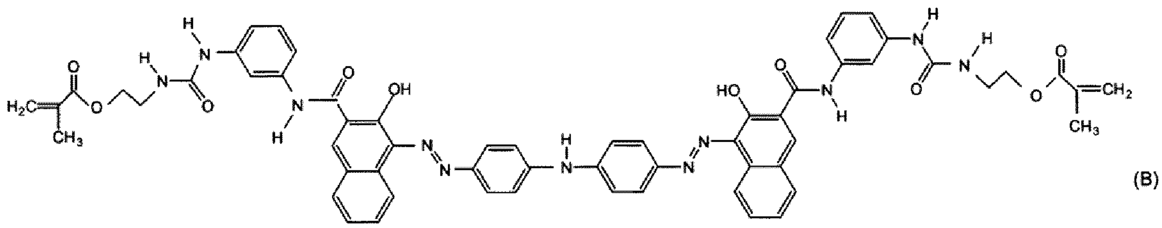

(B)

Examples 19 and 20 and Reference Examples 21 to 24, and Comparative Examples 1 and 2:

Production of Ophthalmic Lens Material

A composition for a contact lens (composition for forming an ophthalmic lens material) was prepared which contains 25 parts by mass of the macromonomer (A1), 25 parts by mass of TRIS, 25 parts by mass of 1,3-MMP, 25 parts by mass of DMAA, 0.4 parts by mass of EDMA, 1.7 parts by mass of HMEPBT, 0.5 parts by mass of TPO, 2 parts by weight of n-PrOH, and each 0.02 parts by mass of the azo colorant shown in Table 2 below. This composition was injected into a mold (made from polypropylene; corresponding to a contact lens having a diameter of about 14 mm and a central thickness of 0.1 mm) having a shape of a contact lens, and then this mold was irradiated with a blue light using a blue fluorescent tube (TL20W03 manufactured by PHILIPS) for 30 min to carry out photopolymerization. The mold

was irradiated from both two faces. After completing the polymerization, a cured matter having the shape of a contact lens was obtained by removing from the mold, and subjected to a surface treatment by further irradiating with plasma (output: 50 W, pressure: 100 Pa) in an oxygen atmosphere for 3 min. Thus treated matter was subjected to a hydration treatment by immersing in a physiological saline solution thereby allowing for absorption of the moisture to obtain contact lenses (ophthalmic lens materials) of Examples 19 to 24 and Comparative Examples 1 and 2.

[Table 2]

| | Azo colorant used |
|---|---|
| Example 19 | azo colorant 1 |
| Example 20 | azo colorant 2 |
| Example 21 | azo colorant 3 |
| Example 22 | azo colorant 9 |
| Example 23 | azo colorant 15 |
| Example 24 | azo colorant 18 |
| Comparative Example 1 | azo colorant A |
| Comparative Example 2 | azo colorant B |

Evaluations The appearance, the specific of lens, the λmax (maximum absorption wavelength) and the elution rate of the colored contact lenses (ophthalmic lens materials) obtained in Examples 19 to 24 and Comparative Example 1 and 2 were evaluated as in the following. The results are shown in Table 3 below.

(1) Appearance

The appearance (color, transparency, presence/absence of alien substances, presence/absence of deformation) of the colored contact lens (ophthalmic lens material) obtained were determined by visual inspection.

(2) Parameters of contact Lenses

The diameter, refractive index, and the curvature radius of the back surface of each of ten pieces of the contact lens were measured in physiological saline adjusted to 20°C, and the lenses were decided as to whether or not the values fell within a specific range.

(3) λmax (maximum absorption wavelength)

A visible absorption spectrum of the contact lens was obtained using an ultraviolet and visible spectrophotometer and an integrating sphere for measuring a contact lens, and λmax (maximum absorption wavelength) derived from the colorant was determined.

(4) Elution Rate

In order to ascertain the elution profile (polymerizability of the colorant) of the colorant from the contact lens, each colored contact lens was immersed in 3 mL of acetone or 2 mL of a wax ester (2-ethylhexyl stearate), which is an eye discharge component, at room temperature for 62 hours, whereby elution of the colorant was allowed. The absorbance at λmax determined in the section (3) above was measured before and after the immersion using the apparatus and the integrating sphere employed in the measurement of the section (3) above, and the elution rate was determined according to the following formula. It is to be noted that five pieces of the contact lens were used in each of the elution test with acetone or wax ester, and the elution rate was determined on each of the pieces according to the following formula. Average values are shown in Tables.

Elution rate (%) = {(absorbance before immersion - absorbance after immersion)/ absorbance before immersion} x 100

It should be noted that a lower elution rate indicates that the colorant is less likely to be eluted from the lens.

[Table 3]

| | | | Example 19 | Example 20 | Example 21 | Example 22 | Example 23 | Example 24 | Comparative Example 1 | Comparative Example 2 |
|---|---|---|---|---|---|---|---|---|---|---|
| Appearance | Color | | pale purple | pale purple | pale purple | pale deep blue | pale deep blue | pale reddish purple | pale purple | pale blue |
| | Transparency | | favorable | favorable | favorable | favorable | favorable | favorable | favorable | somewhat unfavorable |
| | Alien substance | | not found | not found | not found | not found | not found | not found | not found | not found |
| | Deformation | | not found | not found | not found | not found | not found | not found | not found | not found |
| Parameters of the Lenses | Diameter | | within range of Parameter | within range of Parameter | within range of Parameter | within range of Parameter | within range of Parameter | within range of Parameter | within range of Parameter | within range of Parameter |
| | Curvature radius | | within range of Parameter | within range of Parameter | within range of Parameter | within range of Parameter | within range of Parameter | within range of Parameter | within range of Parameter | within range of Parameter |
| | Back vertex power | | within range of Parameter | within range of Parameter | within range of Parameter | within range of Parameter | within range of Parameter | within range of Parameter | within range of Parameter | within range of Parameter |
| λmax (nm) | | | 585 | 580 | 580 | 590 | 605 | 520 | 580 | 625 |
| Elution rate (%) | | acetone | 0.4 | 25.8 | 6.0 | 14.2 | 7.6 | 1.8 | 32.9 | 7.3 |
| | | wax ester | 6.0 | 4.6 | 2.3 | 3.4 | 4.0 | 3.0 | 21.6 | -4.5 |

Examples 25 and 26 and Reference Example 27, and Comparative Example 3: (production of ophthalmic lens material)

A composition for a contact lens (composition for forming an ophthalmic lens material) was prepared which contains 10 parts by mass of the macromonomer (A1), 25 parts by mass of TRIS, 40 parts by mass of N-VP, 25 parts by mass of 2-MTA, 0.3 parts by mass of AMA, 0.4 parts by mass of HMPPO, and each 0.02 parts by mass of the azo colorant shown in Table 4 below. This composition was injected into a mold (made from polypropylene; corresponding to a contact lens having a diameter of about 14 mm and a central thickness of 0.1 mm) having a shape of a contact lens, and then this mold was irradiated with a UV light using a high pressure mercury lamp (2 kW) for 20 min to carry out photopolymerization. The mold was irradiated from only one side corresponding to an FC surface. After completing the polymerization, a cured matter having the shape of a contact lens was removed from the mold, and subjected to a surface treatment by further irradiating with plasma (output: 50 W, pressure: 100 Pa) in an oxygen atmosphere for 3 min. Thus treated matter was subjected to a hydration treatment by immersing in a physiological saline solution thereby allowing for absorption of the moisture to obtain contact lenses (ophthalmic lens materials) of Examples 25 to 27 and Comparative Example 3.

[Table 4]

| | Azo colorant used |
|---|---|
| Example 25 | azo colorant 1 |
| Example 26 | azo colorant 2 |
| Example 27 | azo colorant 3 |
| Comparative Example 3 | azo colorant A |

Evaluations

The appearance, the specific of lens, the λmax (maximum absorption wavelength) and the elution rate of the colored contact lenses (ophthalmic lens materials) obtained in Examples 25 to 27 and Comparative Example 3 were evaluated in accordance with the methods described above. The results are shown in Table 5 below.

[Table 5]

| | | Example 25 | Example 26 | Example 27 | Comparative Example 3 |
|---|---|---|---|---|---|
| Appearance | Color | pale purple | pale purple | pale purple | pale purple |
| | Transparency | favorable | favorable | favorable | favorable |
| | Alien substance | not found | not found | not found | not found |
| | Deformation | not found | not found | not found | not found |
| Parameters of the Lenses | Diameter | within range of Parameter | within range of Parameter | within range of Parameter | within range of Parameter |
| | Curvature Radius | within range of Parameter | within range of Parameter | within range of Parameter | within range of Parameter |
| | Back vertex power | within range of Parameter | within range of Parameter | within range of Parameter | within range of Parameter |
| λmax (nm) | | 555 | 555 | 555 | 555 |
| Elution rate (%) | acetone | 9.9 | 8.6 | 9.5 | 34.8 |
| | wax ester | 10.1 | 18.0 | 26.0 | 37.1 |

As is seen from the results shown in Table 3 and Table 5 above, it is proven that the ophthalmic lens materials (colored contact lenses) of Examples 19 to 27 are superior in elution resistance since the azo colorant used had a plurality of polymerizable group. In addition, due to miscibility of the azo colorant with other monomer of the ophthalmic lens being favorable it is revealed that high transparency can be imparted to ophthalmic lenses.

[INDUSTRIAL APPLICABILITY]

Since the azo colorant of the present invention can strongly bind to other monomer, it can be suitably used as a colorant of ophthalmic lens materials. The ophthalmic lens material can be suitably used for ophthalmic lenses such as e.g., contact lenses, intraocular lens, artificial cornea, cornea onlay and cornea inlay.

## Claims

**1.** A monoazo colorant for coloring an ophthalmic lens represented by a general formula (1):

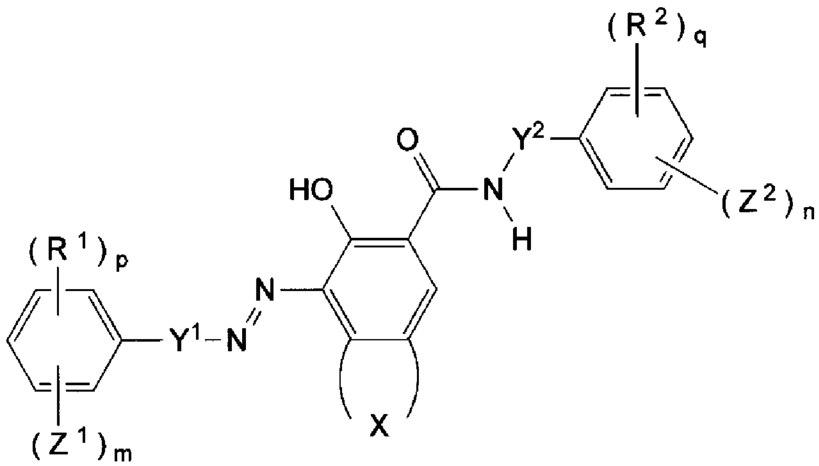

(1)

wherein, X represents a group of atoms that forms a naphthalene ring, an anthracene ring or a benzocarbazole ring together with a benzene ring linking to X; $Y^1$ and $Y^2$ each independently represent a single bond; $Z^1$ and $Z^2$ each independently represent at least one group selected from the group consisting of groups represented by formulae (i) to (vii):

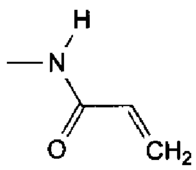

(i)

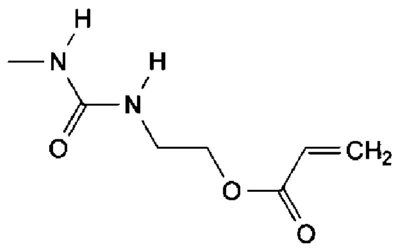

(ii)

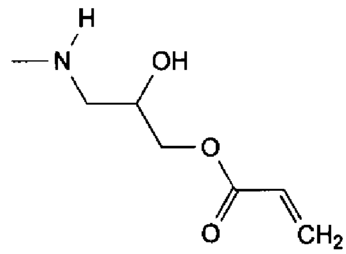

(iii)

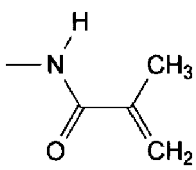

(iv)

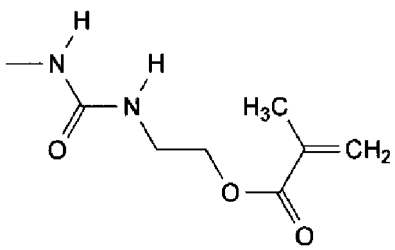

(v)

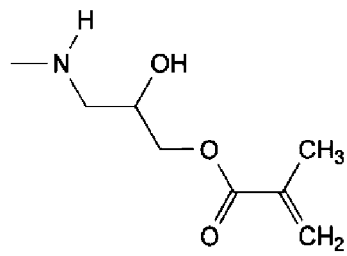

(vi)

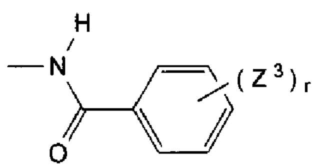

(vii)

wherein, in the formula (vii), $Z^3$ represents at least one group selected from the group consisting of groups represented by the above formulae (i) to (vi);
$R^1$ and $R^2$ each independently represent at least one group selected from the group consisting of an alkyl group having 1 to 5 carbon atoms, and an alkoxy group having 1 to 5 carbon atoms; m, n and r are each independently an integer of 0 to 2, wherein m, n and r are defined such that the total number of the groups represented by the above formulae (i) to (vi) is no less than 2 and no greater than 4; and p is an integer of 2; and q is an integer of 0 to 5, wherein the sum of p and m, and the sum of q and n are each no greater than 5, wherein in a case where m,

n, r, p and q are each no less than 2, a plurality of $Z^1$s, $Z^2$s, $Z^3$s, $R^1$s and $R^2$s are each independently as defined above.

2. An ophthalmic lens material produced from a composition comprising the monoazo colorant according to claim 1 and a monomer for an ophthalmic lens having a polymerizable group, which comprises a monomer having a polydimethylsiloxane structure, a monomer for an ophthalmic lens that is hydrophilic, a nonhydrous monomer for an ophthalmic lens or a combination thereof, thereby comprising a copolymer of the monoazo colorant and the monomer for an ophthalmic lens.

3. The ophthalmic lens material according to claim 2, wherein the monomer for an ophthalmic lens comprises a compound having a polydimethylsiloxane structure.

4. The ophthalmic lens material according to claim 3, wherein the polydimethylsiloxane structure and the polymerizable group are present via a urethane bond in the monomer for an ophthalmic lens.

5. The ophthalmic lens material according to any one of claims 2 to 4, wherein the monomer for an ophthalmic lens comprises a (meth)acrylic acid derivative, a styrene derivative, an allyl group-containing compound, a vinyl group-containing compound, an exo-methylene group-containing compound or a combination thereof.

6. The ophthalmic lens material according to any one of claims 2 to 5, wherein the monomer for an ophthalmic lens is hydrophilic.

7. A method for producing an ophthalmic lens material, the method comprising the steps of:

preparing a composition for an ophthalmic lens material comprising the monoazo colorant according to claim 1, and a monomer for an ophthalmic lens having a polymerizable group;
transferring the composition into a mold;
curing the composition in the mold by heating, irradiation with an ultraviolet ray, a visible ray or a combination of an ultraviolet ray and a visible ray, or both the heating and the irradiation to obtain a cured matter; and
releasing the cured matter.

8. The method for producing an ophthalmic lens material according to claim 7, wherein the releasing step is carried out without using a medium, while maintaining the cured matter in a dry state.

9. The method for producing an ophthalmic lens material according to claim 7 or 8, further comprising the steps of:

hydrating the cured matter; and
removing an unreacted material from the cured matter hydrated.

10. The method for producing an ophthalmic lens material according to claim 9, wherein the hydrating step and the removing step are concomitantly carried out by immersing the cured matter obtained through the releasing step in water, physiological saline, phosphoric acid buffer or boric acid buffer.

11. The method for producing an ophthalmic lens material according to any one of claims 7 to 10, wherein the monomer for an ophthalmic lens comprises a compound having a polydimethylsiloxane structure.

12. The method for producing an ophthalmic lens material according to any one of claims 7 to 11, wherein the monomer for an ophthalmic lens is hydrophilic.

13. The method for producing an ophthalmic lens material according to any one of claims 7 to 12, wherein the method further comprises the step of:
subjecting the cured matter released to a surface treatment.

14. The method for producing an ophthalmic lens material according to claim 13, wherein the step of subjecting the cured matter released to a surface treatment comprises:

exposing the cured matter to a plasma discharging atmosphere;
subjecting a surface of the cured matter to surface graft polymerization; or
irradiating the cured matter with an ultraviolet ray or a radioactive ray.

**15.** An ophthalmic lens colored with the monoazo colorant according to claim 1.

**Patentansprüche**

**1.** Monoazofarbstoff zum Färben eines Brillenglases, der durch die allgemeine Formel (1) dargestellt wird:

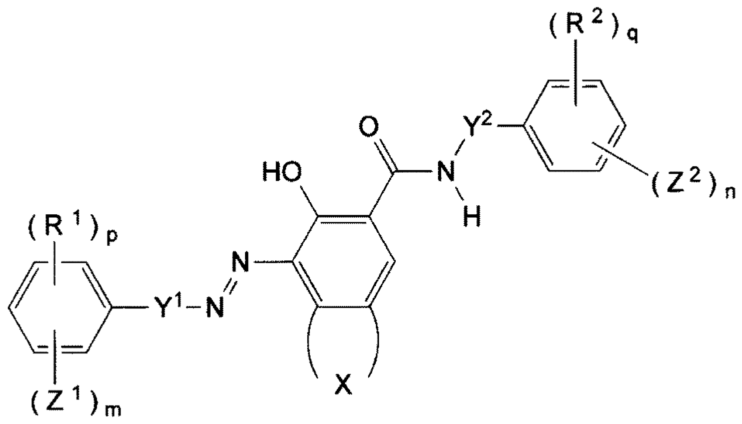

(1)

wobei X für eine Gruppe von Atomen steht, die zusammen mit dem an X gebundenen Benzolring einen Naphthalinring, einen Anthracenring oder einen Benzocarbazolring bilden; $Y^1$ und $Y^2$ jeweils unabhängig für eine Einfachbindung stehen; $Z^1$ und $Z^2$ jeweils unabhängig für wenigstens eine Gruppe stehen, die aus der Gruppe ausgewählt ist, welche aus Gruppen besteht, die durch die Formeln (i) bis (vii) dargestellt werden:

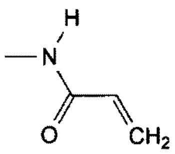

(i)

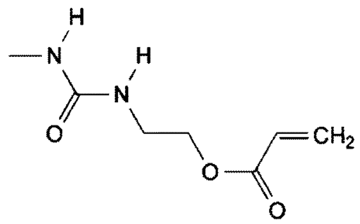

(ii)

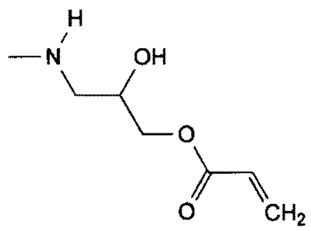

(iii)

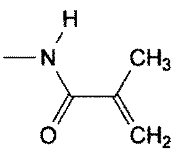

(iv)

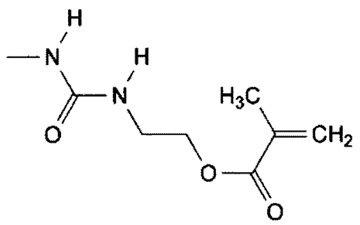

(v)

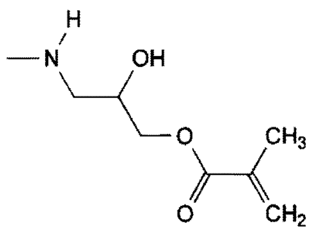

(vi)

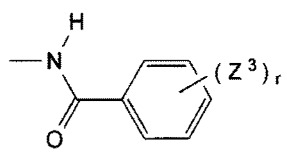

(vii)

wobei in Formel (vii) $Z^3$ für wenigstens eine Gruppe stehen, die aus der Gruppe ausgewählt ist, welche aus Gruppen besteht, die durch die Formeln (i) bis (vi) dargestellt werden;
$R^1$ und $R^2$ jeweils unabhängig für wenigstens eine Gruppe stehen, die aus der Gruppe ausgewählt ist, welche aus

einer Alkylgruppe mit 1 bis 5 Kohlenstoffatomen und einer Alkoxygruppe mit 1 bis 5 Kohlenstoffatomen besteht; m, n und r jeweils unabhängig eine ganze Zahl von 0 bis 2 sind, wobei m, n und r so definiert sind, dass die Gesamtzahl der durch die obigen Formeln (i) bis (vi) dargestellten Gruppen nicht kleiner als 2 und nicht größer als 4 ist; und p die ganze Zahl 2 ist; und q eine ganze Zahl von 0 bis 5 ist, wobei die Summe von p und m und die Summe von q und n jeweils nicht größer als 5 sind, wobei in einem Fall, in dem m, n, r, p und q jeweils nicht kleiner als 2 sind, eine Vielzahl von $Z^1$, $Z^2$, $Z^3$, $R^1$ und $R^2$ jeweils unabhängig wie oben definiert sind.

**2.** Brillenglasmaterial, hergestellt aus einer Zusammensetzung, die den Monoazofarbstoff gemäß Anspruch 1 und ein Monomer für ein Brillenglas mit einer polymerisierbaren Gruppe umfasst, welches ein Monomer mit einer Polydimethylsiloxan-Struktur, ein Monomer für ein Brillenglas, das hydrophil ist, ein wasserfreies Monomer für ein Brillenglas oder eine Kombination davon umfasst, wodurch sie ein Copolymer des Monoazofarbstoffs und das Monomer für ein Brillenglas umfasst.

**3.** Brillenglasmaterial gemäß Anspruch 2, wobei das Monomer für ein Brillenglas eine Verbindung mit einer Polydimethylsiloxan-Struktur umfasst.

**4.** Brillenglasmaterial gemäß Anspruch 3, wobei die Polydimethylsiloxan-Struktur und die polymerisierbare Gruppe über eine Urethanbindung in dem Monomer für ein Brillenglas vorhanden sind.

**5.** Brillenglasmaterial gemäß einem der Ansprüche 2 bis 4, wobei das Monomer für ein Brillenglas ein (Meth)acrylsäure-Derivat, ein Styrolderivat, eine Allylgruppen enthaltende Verbindung, eine Vinylgruppen enthaltende Verbindung, eine exo-Methylengruppen enthaltende Verbindung oder eine Kombination davon umfasst.

**6.** Brillenglasmaterial gemäß einem der Ansprüche 2 bis 5, wobei das Monomer für ein Brillenglas hydrophil ist.

**7.** Verfahren zur Herstellung eines Brillenglasmaterials, wobei das Verfahren die folgenden Schritte umfasst:

Herstellen einer Zusammensetzung für ein Brillenglasmaterial, die den Monoazofarbstoff gemäß Anspruch 1 und ein Monomer für ein Brillenglas mit einer polymerisierbaren Gruppe umfasst;
Überführen der Zusammensetzung in eine Form;
Härten der Zusammensetzung in der Form durch Erhitzen, Bestrahlen mit Ultraviolettstrahlung, sichtbarer Strahlung oder einer Kombination aus Ultraviolettstrahlung und sichtbarer Strahlung oder sowohl durch Erhitzen als auch durch Bestrahlen, wobei man eine gehärtete Substanz erhält; und Entnehmen der gehärteten Substanz.

**8.** Verfahren zur Herstellung eines Brillenglasmaterials gemäß Anspruch 7, wobei der Schritt des Entnehmens durchgeführt wird, ohne ein Medium zu verwenden, während die gehärtete Substanz in einem trockenen Zustand gehalten wird.

**9.** Verfahren zur Herstellung eines Brillenglasmaterials gemäß Anspruch 7 oder 8, weiterhin umfassend die Schritte:

Hydratisieren der gehärteten Substanz; und
Entfernen von nicht umgesetztem Material aus der hydratisierten gehärteten Substanz.

**10.** Verfahren zur Herstellung eines Brillenglasmaterials gemäß Anspruch 9, wobei der Schritt des Hydratisierens und der Schritt des Entfernens gleichzeitig durchgeführt werden, indem man die durch den Schritt des Entnehmens erhaltene gehärtete Substanz in Wasser, physiologische Kochsalzlösung, Phosphorsäurepuffer oder Borsäurepuffer eintaucht.

**11.** Verfahren zur Herstellung eines Brillenglasmaterials gemäß einem der Ansprüche 7 bis 10, wobei das Monomer für ein Brillenglas eine Verbindung mit einer Polydimethylsiloxan-Struktur umfasst.

**12.** Verfahren zur Herstellung eines Brillenglasmaterials gemäß einem der Ansprüche 7 bis 11, wobei das Monomer für ein Brillenglas hydrophil ist.

**13.** Verfahren zur Herstellung eines Brillenglasmaterials gemäß einem der Ansprüche 7 bis 12, wobei das Verfahren weiterhin den folgenden Schritt umfasst:
Durchführen einer Oberflächenbehandlung mit der entnommenen gehärteten Substanz.

**14.** Verfahren zur Herstellung eines Brillenglasmaterials gemäß Anspruch 13, wobei der Schritt des Durchführens einer Oberflächenbehandlung mit der entnommenen gehärteten Substanz Folgendes umfasst:

Einwirkenlassen einer Plasmaentladungsatmosphäre auf die gehärtete Substanz;
Durchführen einer Oberflächenpfropfpolymerisation mit einer Oberfläche der gehärteten Substanz; oder
Bestrahlen der gehärteten Substanz mit Ultraviolettstrahlung oder radioaktiver Strahlung.

**15.** Brillenglas, gefärbt mit dem Monoazofarbstoff gemäß Anspruch 1.

## Revendications

**1.** Colorant monoazo pour colorer une lentille ophtalmique, représenté par une formule générale (1) :

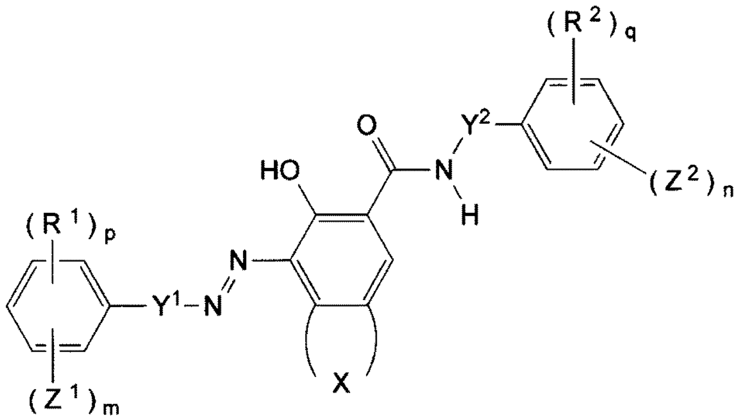

(1)

dans lequel X représente un groupe d'atomes formant un cycle naphtalène, un cycle anthracène, ou un cycle benzocarbazole, conjointement avec un cycle benzène relié à X, $Y^1$ et $Y^2$ représentent chacun indépendamment une liaison simple, $Z^1$ et $Z^2$ représentent chacun indépendamment au moins un groupe choisi dans le groupe consistant en des groupes représentés par les formules (i) à (vii) :

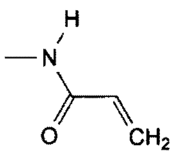

(i)

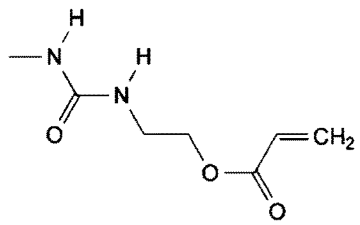

(ii)

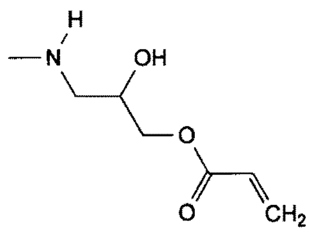

(iii)

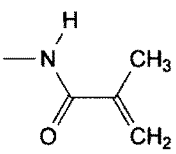

(iv)

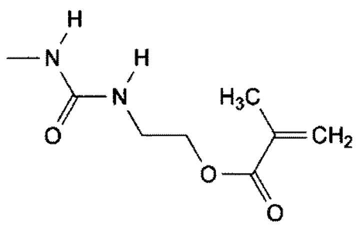

(v)

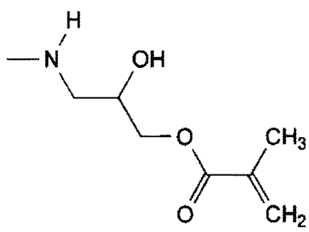

(vi)

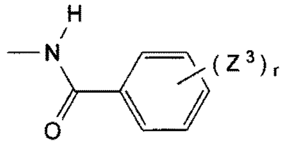

( vii )

dans lequel, dans la formule (vii), $Z^3$ représente au moins un groupe choisi dans le groupe consistant en des groupes représentés par les formules (i) à (vi) ci-dessus,
$R^1$ et $R^2$ représentent chacun indépendamment au moins un groupe choisi dans le groupe consistant en un groupe alkyle ayant 1 à 5 atomes de carbone, et un groupe alkoxy ayant 1 à 5 atomes de carbone, m, n et r sont chacun indépendamment un nombre entier de 0 à 2, dans lequel m, n et r sont définis de manière que le nombre total des groupes représentés par les formules (i) à (vi) ci-dessus n'est pas inférieur à 2 et n'est pas supérieur à 4, et p est le nombre entier 2, et q est un nombre entier de 0 à 5, dans lequel la somme de p et m, et la somme de q et n ne sont pas supérieures chacune à 5, dans lequel, dans le cas où m, n, r, p et q ne sont pas inférieures chacun à 2, une pluralité de $Z^1$s, $Z^2$s, $Z^3$s, $R^1$s et $R^2$s sont chacun indépendamment tels que définis ci-dessus.

2. Matériau de lentille ophtalmique, préparé à partir d'une composition comprenant le colorant monoazo selon la revendication 1 et un monomère pour une lentille ophtalmique ayant un groupe polymérisable, comprenant un monomère ayant une structure polydiméthylsiloxane, un monomère pour une lentille ophtalmique qui est hydrophile, un monomère anhydre pour une lentille ophtalmique, ou une combinaison de ceux-ci, ainsi comprenant un copolymère du colorant monoazo et le monomère pour une lentille ophtalmique.

3. Matériau de lentille ophtalmique selon la revendication 2, dans lequel ledit monomère pour une lentille ophtalmique comprend un composé ayant une structure polydiméthylsiloxane.

4. Matériau de lentille ophtalmique selon la revendication 3, dans lequel la structure polydiméthylsiloxane et le groupe polymérisable sont présents au moyen d'une liaison uréthane dans le monomère pour une lentille ophtalmique.

5. Matériau de lentille ophtalmique selon l'une quelconque des revendications 2 à 4, dans lequel ledit monomère pour une lentille ophtalmique comprend un dérivé d'acide (méth)acrylique, un dérivé de styrène, un composé contenant un groupe allyle, un composé contenant un groupe vinyle, un composé contenant un groupe exo-méthylène, ou une combinaison de ceux-ci.

6. Matériau de lentille ophtalmique selon l'une quelconque des revendications 2 à 5, dans lequel ledit monomère pour une lentille ophtalmique est hydrophile.

7. Procédé pour préparer un matériau de lentille ophtalmique, ledit procédé comprenant les étapes consistant à

   préparer une composition pour un matériau de lentille ophtalmique, comprenant le colorant monoazo selon la revendication 1 et un monomère pour une lentille ophtalmique ayant un groupe polymérisable,
   transférer la composition dans un moule,
   durcir la composition dans le moule par chauffage, par rayonnement aux rayons ultraviolets, aux rayons visibles, ou à la combinaison de rayons ultraviolets et rayons visibles, ou par les deux, le chauffage et le rayonnement, pour obtenir une matière durcie, et
   démouler la matière durcie.

8. Procédé pour préparer un matériau de lentille ophtalmique selon la revendication 7, dans lequel l'étape de démoulage est effectuée sans utiliser un milieu, tout en maintenant la matière durcie dans un état sec.

9. Procédé pour préparer un matériau de lentille ophtalmique selon la revendication 7 ou 8, comprenant en outre les étapes consistant à

   hydratiser la matière durcie, et
   enlever un matériau n'ayant pas réagi de la matière durcie hydratisée.

10. Procédé pour préparer un matériau de lentille ophtalmique selon la revendication 9, dans lequel l'étape consistant

à hydratiser et l'étape consistant à enlever sont effectuées simultanément, par l'immersion de la matière durcie obtenue par l'étape de démoulage dans de l'eau, une solution saline normale, un tampon d'acide phosphorique, ou un tampon d'acide borique.

**11.** Procédé pour préparer un matériau de lentille ophtalmique selon l'une quelconque des revendications 7 à 10, dans lequel ledit monomère pour une lentille ophtalmique comprend un composé ayant une structure polydiméthylsiloxane.

**12.** Procédé pour préparer un matériau de lentille ophtalmique selon l'une quelconque des revendications 7 à 11, dans lequel ledit monomère pour une lentille ophtalmique est hydrophile.

**13.** Procédé pour préparer un matériau de lentille ophtalmique selon l'une quelconque des revendications 7 à 12, dans lequel le procédé comprend en outre l'étape consistant à
soumettre la matière durcie démoulée à un traitement de surface.

**14.** Procédé pour préparer un matériau de lentille ophtalmique selon la revendication 13, dans lequel l'étape consistant à soumettre la matière durcie démoulée à un traitement de surface comprend les étapes consistant à

exposer la matière durcie à une atmosphère de décharge de plasma,
soumettre une surface de la matière durcie à une polymérisation de greffe superficielle, ou
irradier la matière durcie avec un rayonnement ultraviolet ou un rayonnement radioactif.

**15.** Lentille ophtalmique colorée avec un colorant monoazo selon la revendication 1.

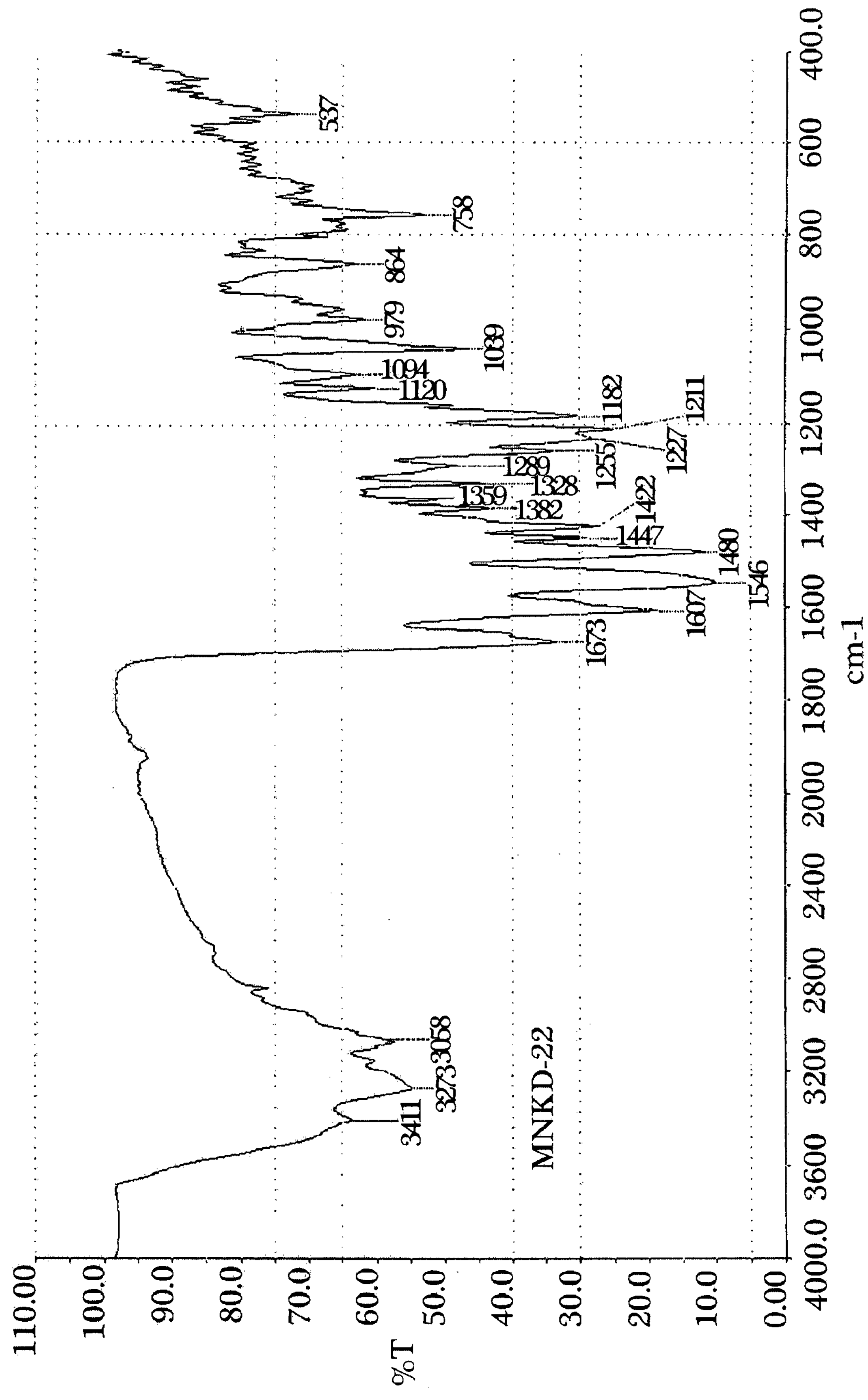
%T
110.00
100.0
90.0
80.0
70.0
60.0
50.0
40.0
30.0
20.0
10.0
0.00
4000.0
3600
3200
2800
2400
2000
1800
1600
1400
1200
1000
800
600
400.0
cm-1
MNKD-22
3411
3273
3058
1673
1607
1546
1480
1447
1422
1382
1359
1328
1289
1255
1227
1211
1182
1120
1094
1039
979
864
758
537

FIG. 1

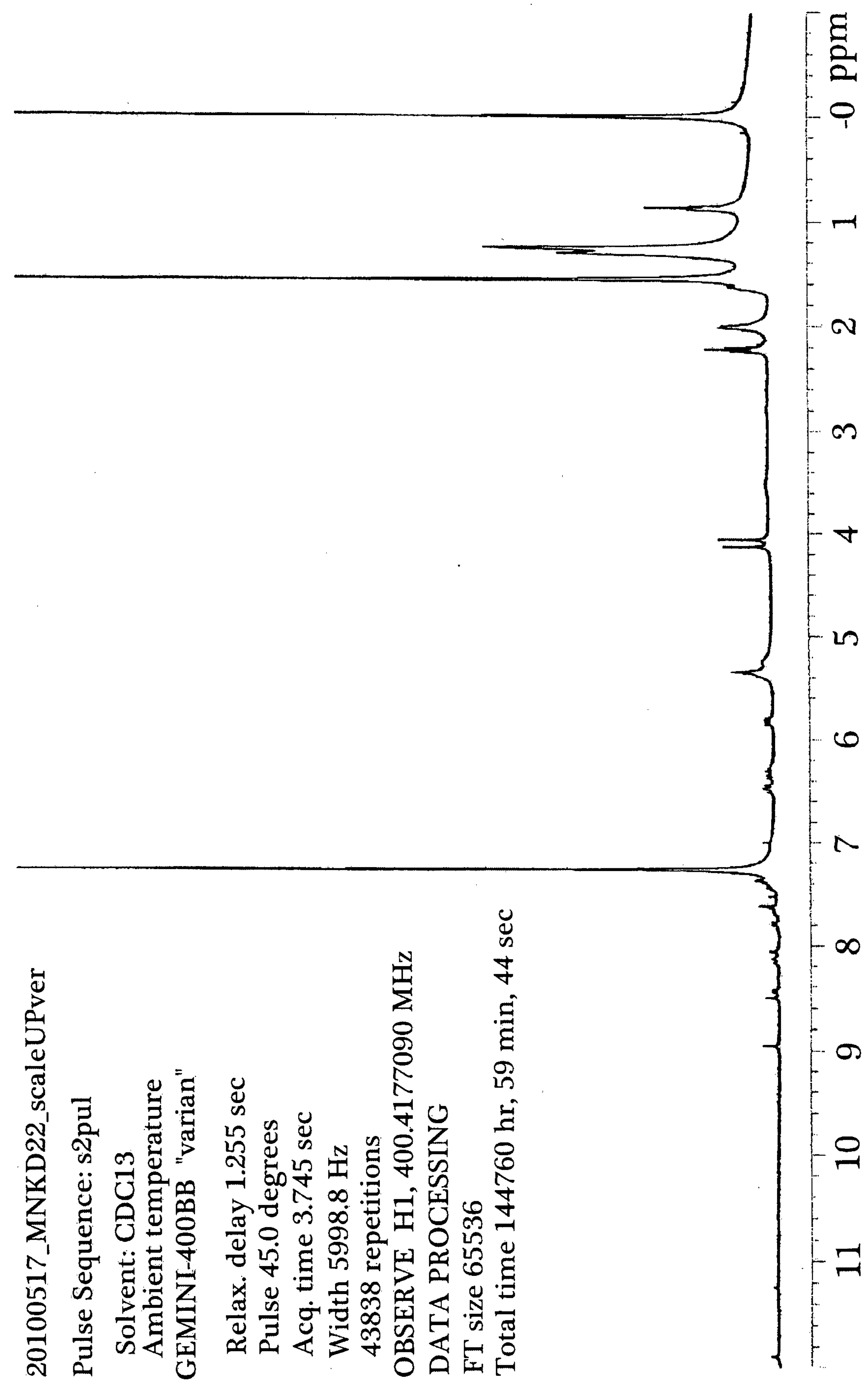
20100517_MNKD22_scaleUPver
Pulse Sequence: s2pul
Solvent: CDC13
Ambient temperature
GEMINI-400BB "varian"
Relax. delay 1.255 sec
Pulse 45.0 degrees
Acq. time 3.745 sec
Width 5998.8 Hz
43838 repetitions
OBSERVE H1, 400.4177090 MHz
DATA PROCESSING
FT size 65536
Total time 144760 hr, 59 min, 44 sec
11
10
9
8
7
6
5
4
3
2
1
-0 ppm

FIG. 2

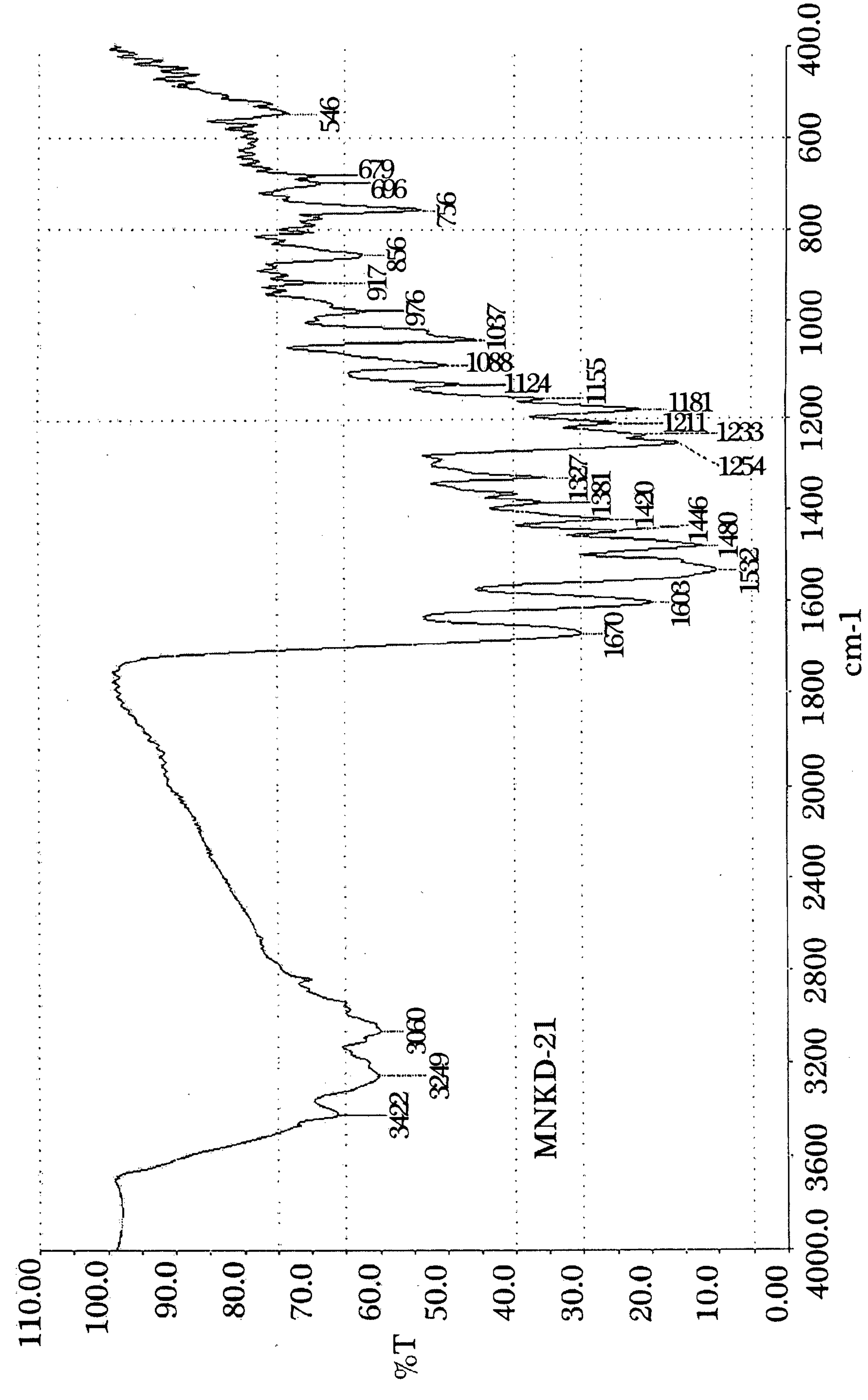
MNKD-21
%T
cm-1
110.00
100.0
90.0
80.0
70.0
60.0
50.0
40.0
30.0
20.0
10.0
0.00
4000.0
3600
3200
2800
2400
2000
1800
1600
1400
1200
1000
800
600
400.0
3422
3249
3060
1670
1603
1532
1480
1446
1420
1381
1327
1254
1233
1211
1181
1155
1124
1088
1037
976
917
856
756
696
679
546

F I G . 3

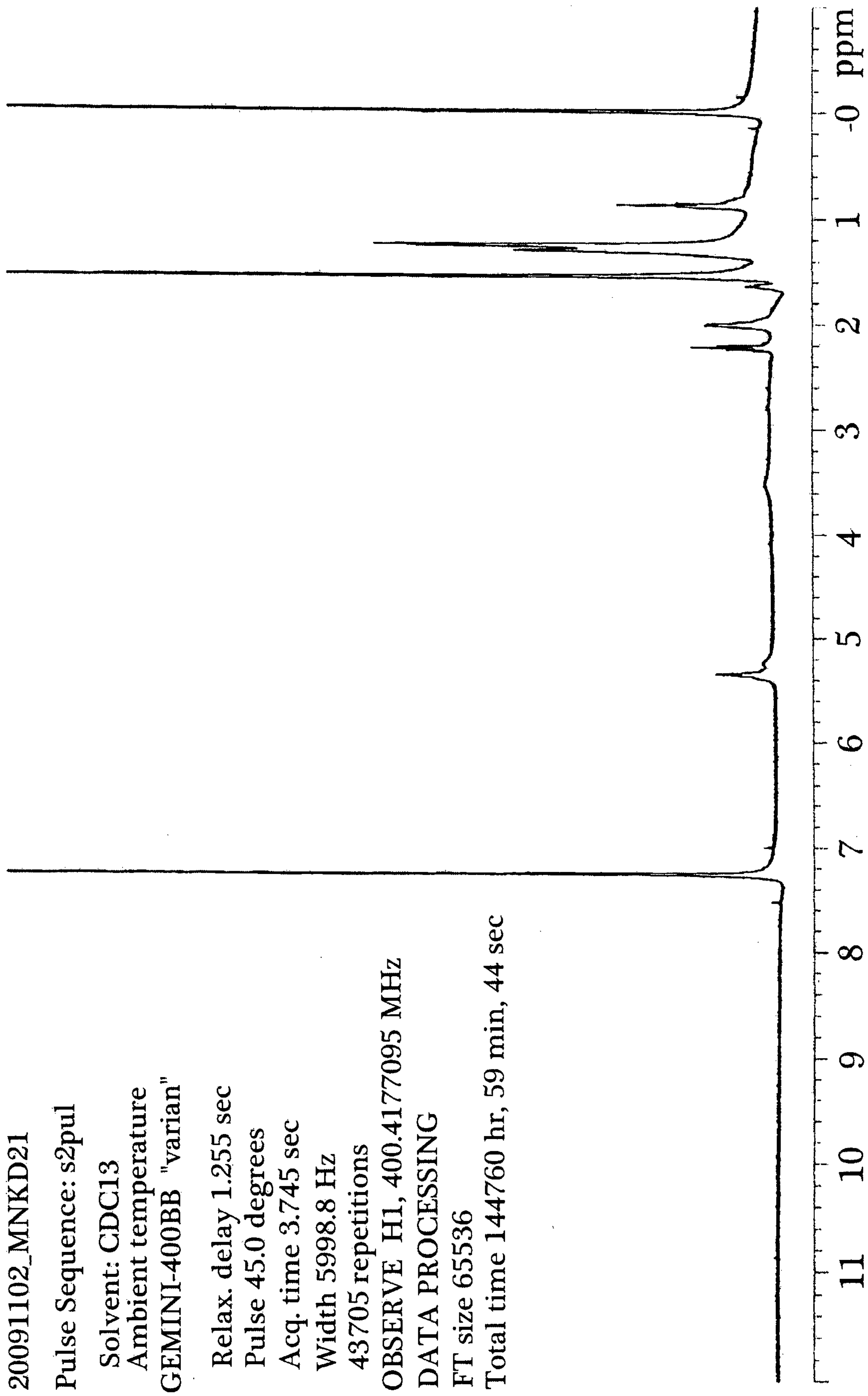
20091102_MNKD21
Pulse Sequence: s2pul
Solvent: CDC13
Ambient temperature
GEMINI-400BB "varian"
Relax. delay 1.255 sec
Pulse 45.0 degrees
Acq. time 3.745 sec
Width 5998.8 Hz
43705 repetitions
OBSERVE H1, 400.4177095 MHz
DATA PROCESSING
FT size 65536
Total time 144760 hr, 59 min, 44 sec
11
10
9
8
7
6
5
4
3
2
1
-0
ppm

FIG. 4

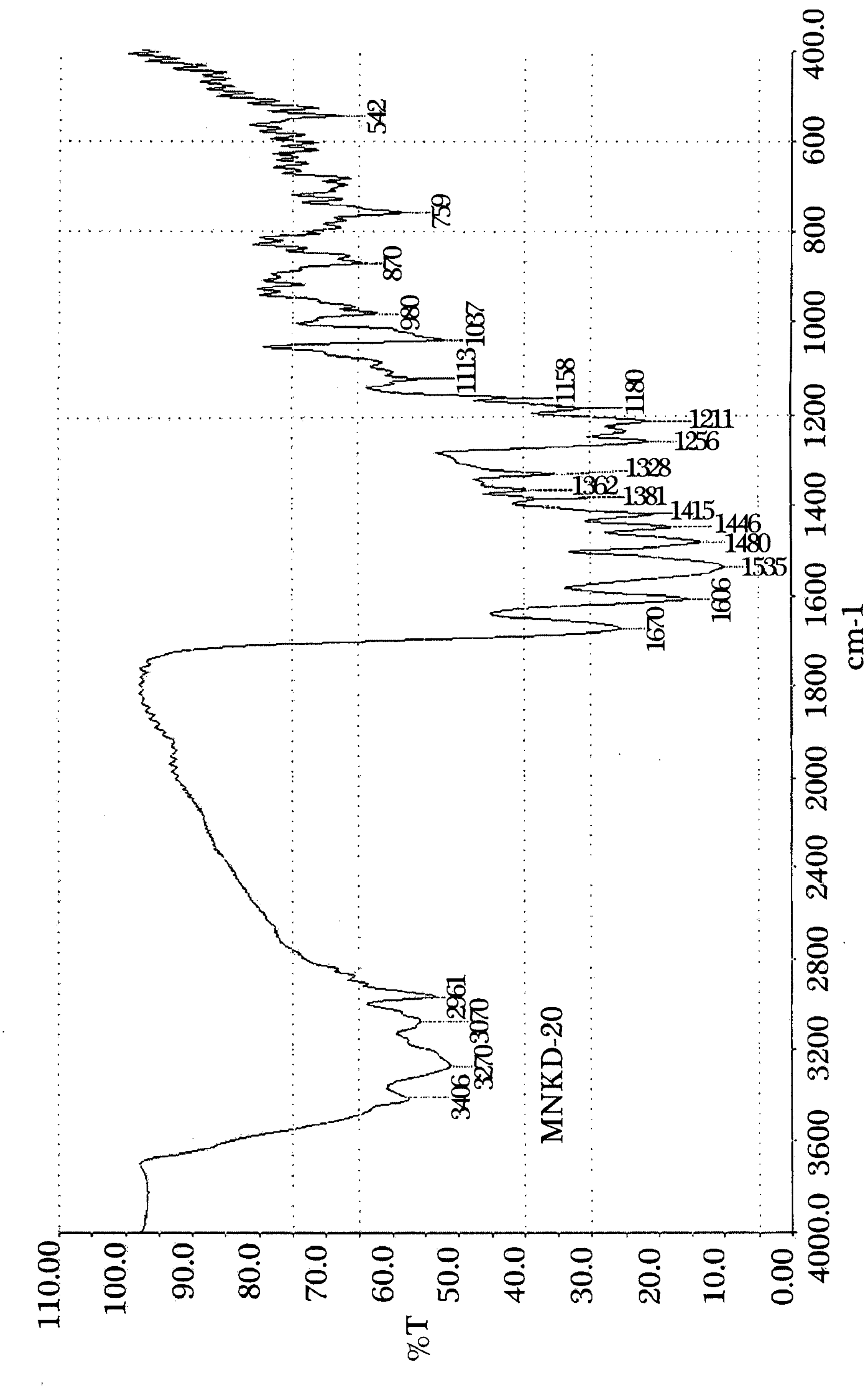
%T
110.00
100.0
90.0
80.0
70.0
60.0
50.0
40.0
30.0
20.0
10.0
0.00
4000.0
3600
3200
2800
2400
2000
1800
1600
1400
1200
1000
800
600
400.0
cm-1
MNKD-20
3406
3270
3070
2961
1670
1606
1535
1480
1446
1415
1381
1362
1328
1256
1211
1180
1158
1113
1037
980
870
759
542

F I G . 5

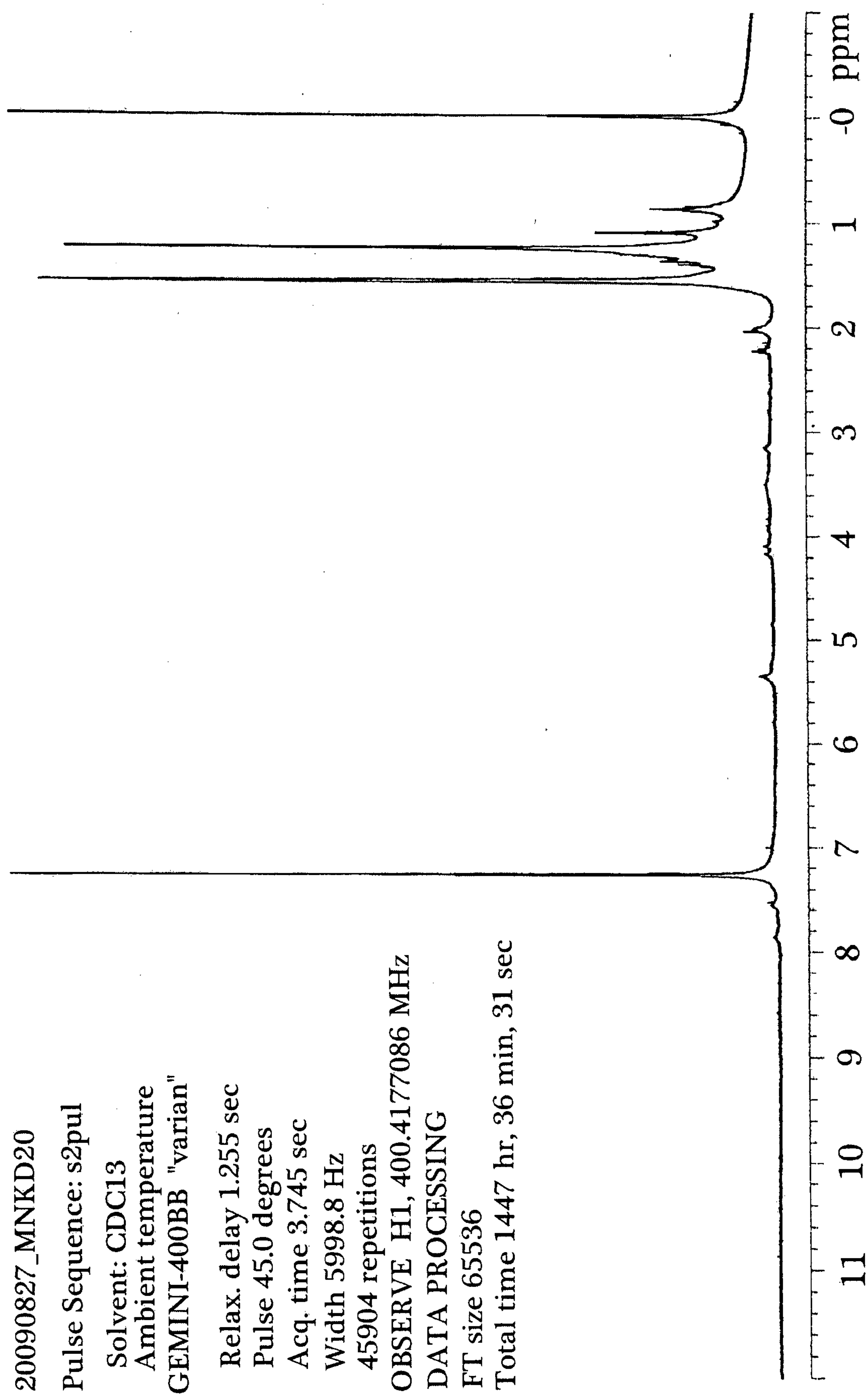
20090827_MNKD20
Pulse Sequence: s2pul
Solvent: CDC13
Ambient temperature
GEMINI-400BB "varian"
Relax. delay 1.255 sec
Pulse 45.0 degrees
Acq. time 3.745 sec
Width 5998.8 Hz
45904 repetitions
OBSERVE H1, 400.4177086 MHz
DATA PROCESSING
FT size 65536
Total time 1447 hr, 36 min, 31 sec
11
10
9
8
7
6
5
4
3
2
1
-0
ppm

F I G. 6

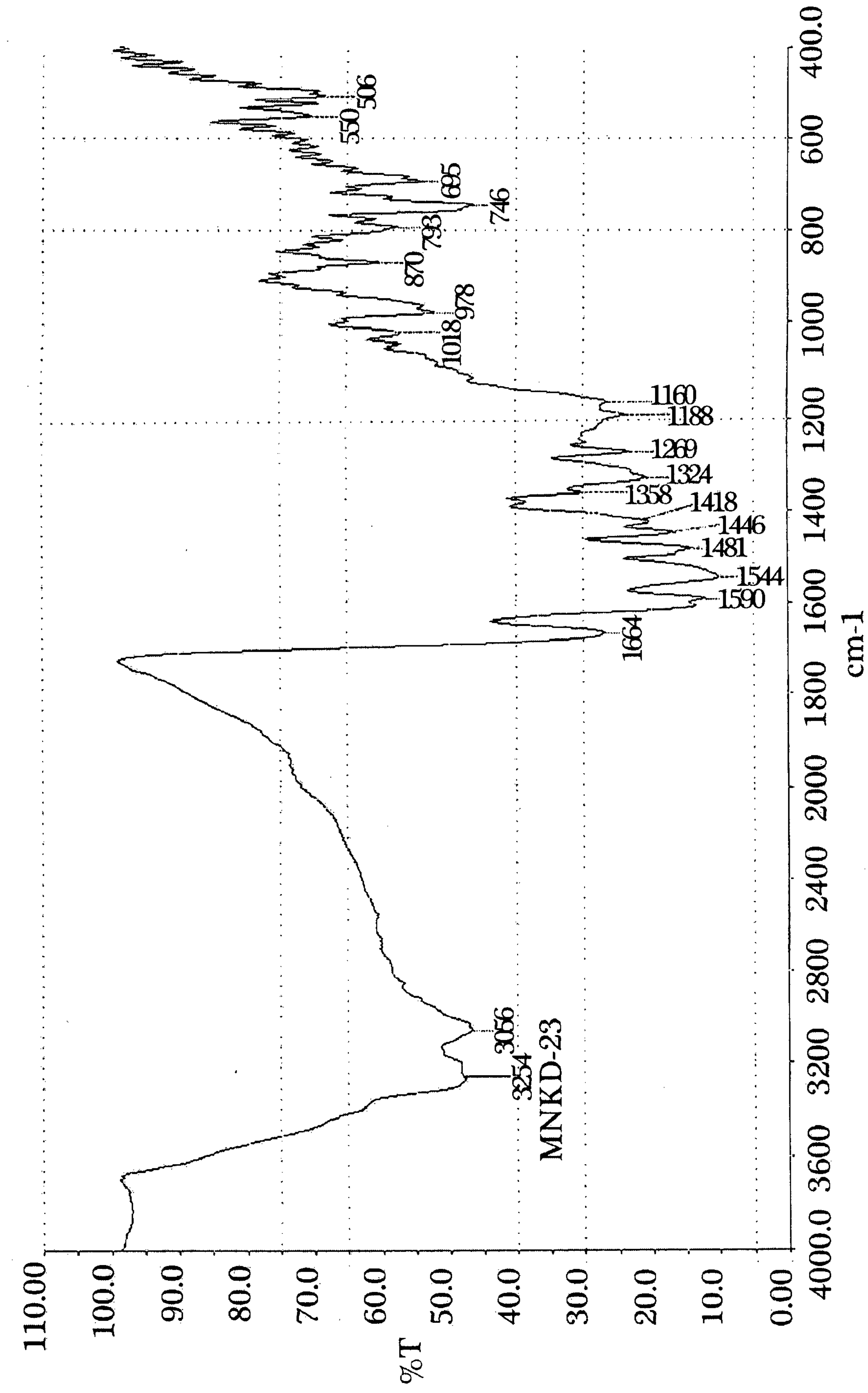

%T
110.00
100.0
90.0
80.0
70.0
60.0
50.0
40.0
30.0
20.0
10.0
0.00
4000.0
3600
3200
2800
2400
2000
1800
1600
1400
1200
1000
800
600
400.0
cm-1
MNKD-23
3254
3056
1664
1590
1544
1481
1446
1418
1358
1324
1269
1188
1160
1018
978
870
793
746
695
550
506


F I G . 7

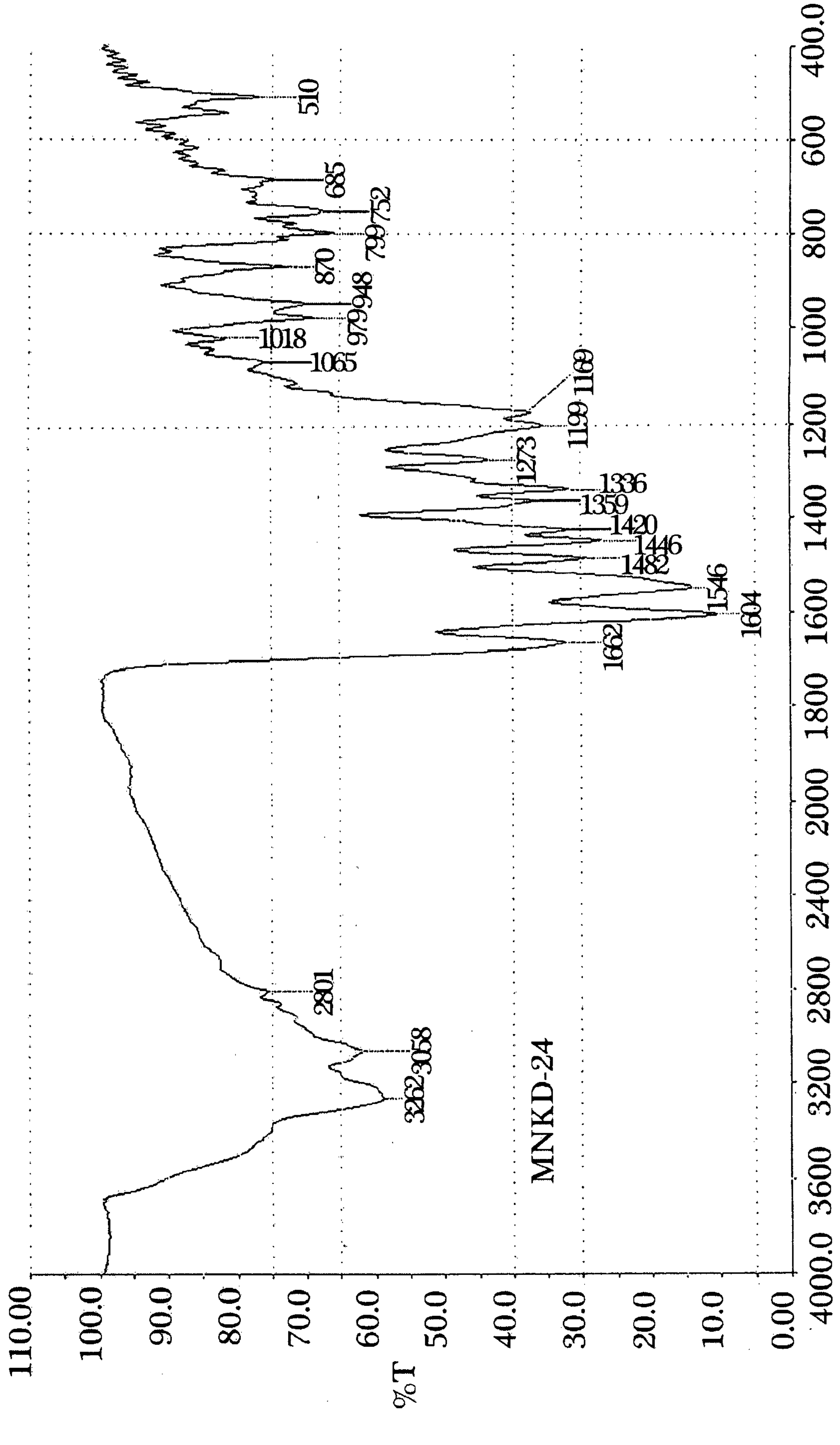
%T
110.00
100.0
90.0
80.0
70.0
60.0
50.0
40.0
30.0
20.0
10.0
0.00
4000.0
3600
3200
2800
2400
2000
1800
1600
1400
1200
1000
800
600
400.0
cm-1
MNKD-24
3262
3058
2801
1662
1604
1546
1482
1446
1420
1359
1336
1273
1199
1169
1065
1018
979
948
870
799
752
685
510

F I G . 8

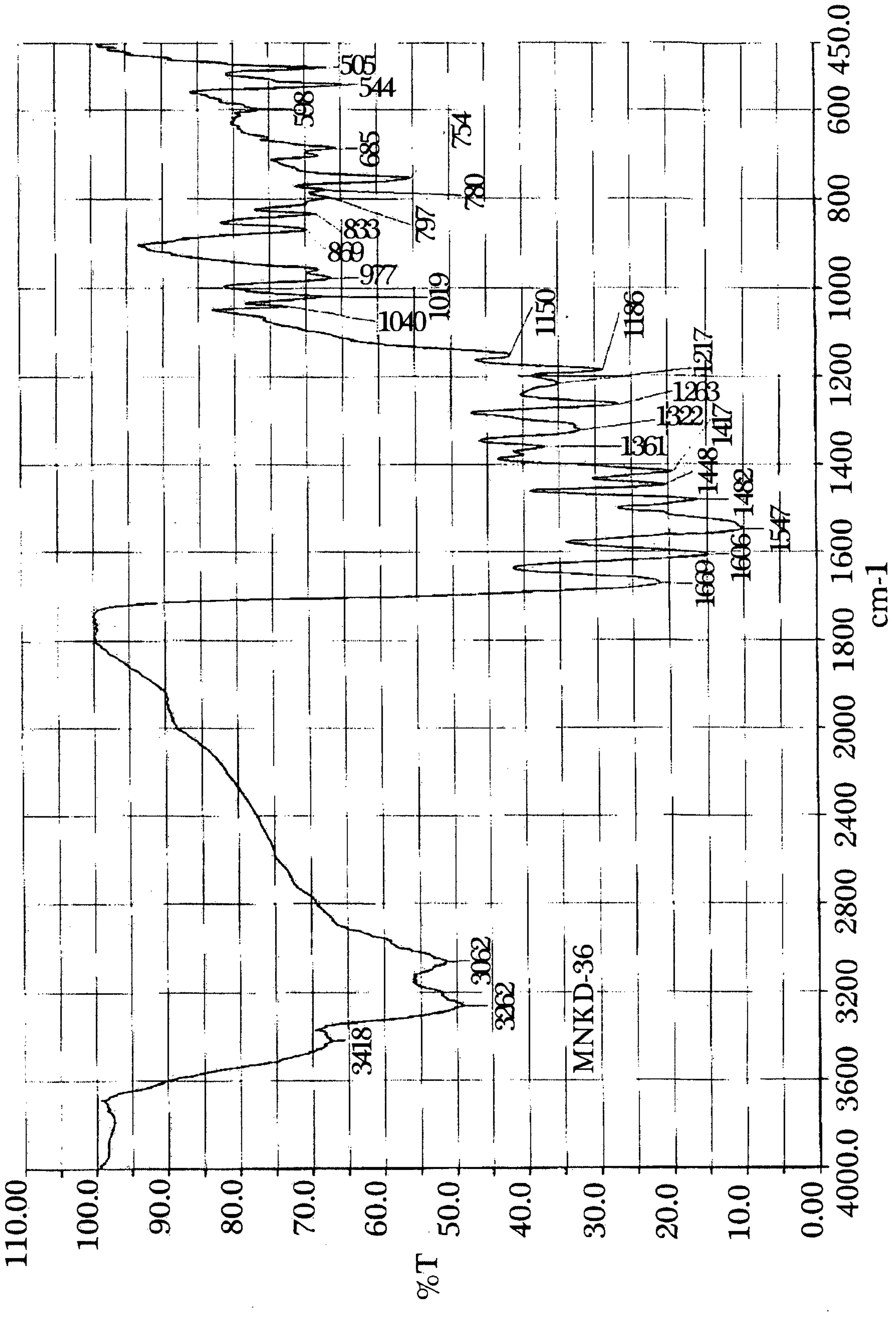
%T
110.00
100.0
90.0
80.0
70.0
60.0
50.0
40.0
30.0
20.0
10.0
0.00
4000.0
3600
3200
2800
2400
2000
1800
1600
1400
1200
1000
800
600
450.0
cm-1
MNKD-36
3418
3262
3062
1660
1606
1547
1482
1448
1417
1361
1322
1263
1217
1186
1150
1040
1019
977
869
833
797
780
754
685
598
544
505

F I G. 9

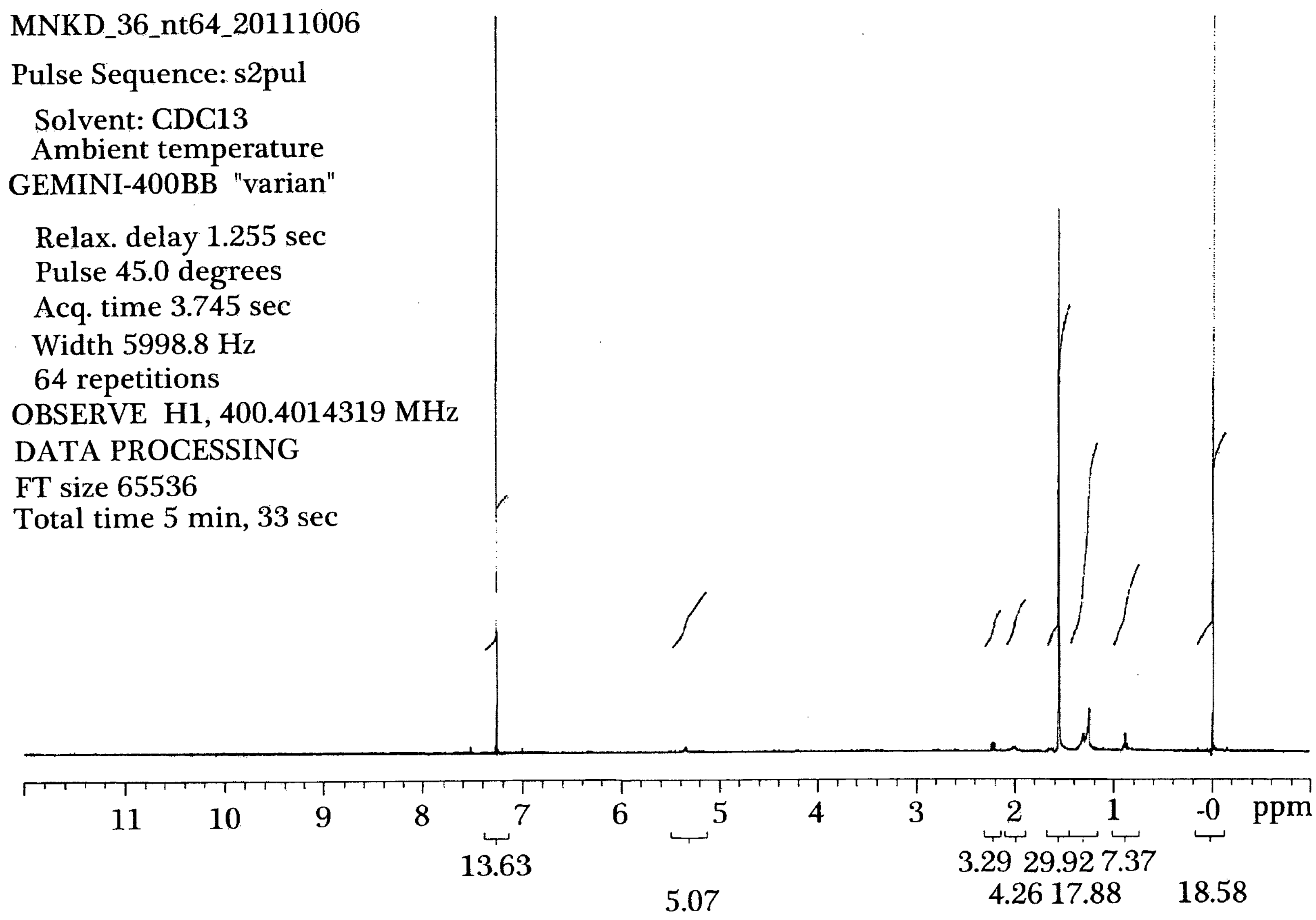
MNKD_36_nt64_20111006
Pulse Sequence: s2pul
Solvent: CDC13
Ambient temperature
GEMINI-400BB "varian"
Relax. delay 1.255 sec
Pulse 45.0 degrees
Acq. time 3.745 sec
Width 5998.8 Hz
64 repetitions
OBSERVE H1, 400.4014319 MHz
DATA PROCESSING
FT size 65536
Total time 5 min, 33 sec
11
10
9
8
7
6
5
4
3
2
1
-0
ppm
13.63
5.07
3.29
4.26
29.92
17.88
7.37
18.58

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007505171 W
- JP 2003084242 A
- JP 2009044853 W
- WO 2006057840 A2
- US 20090240329 A1
- JP 2005116728 W
- JP 2004063795 W
- WO 2004063795 A

### Non-patent literature cited in the description

- **S.S. ACHI ; T.W.J. APPERLEY.** Macromolecular Azo Pigments. *Deyes and Pigments,* 1986, vol. 7, 319-340